# EUROPEAN PATENT SPECIFICATION

(11) **EP 0 638 079 B1**
(45) Date of publication and mention of the grant of the patent: **15.12.1999**
(21) Application number: 93910852.8
(22) Date of filing: 28.04.1993
(51) Int. Cl.: C07D 471/04, A61K 31/505

(54) **DEAZAAMINOPTERINS FOR TREATMENT OF INFLAMMATION**
DEAZAAMINOPTERINE FÜR BEHANDLUNG VON ENTZÜNDUNGEN
DESAZAAMINOPTERINES POUR LE TRAITEMENT DES INFLAMMATIONS

(30) Priority: 29.04.1992 US 875779; 26.01.1993 US 8919
(43) Date of publication of application: 15.02.1995
(73) Proprietor: SRI INTERNATIONAL, Menlo Park, California 94025-3493 (US)
(72) Inventor: PIPER, James, R., Birmingham, AL 35243 (US); DEGRAW, Joseph, I., Sunnyvale, CA 94087 (US); COLWELL, William, T., Menlo Park, CA 94025 (US); SIROTNAK, Francis, M., New York, NY 10021 (US); SMITH, R., Lane, Palo Alto, CA 94306-2618 (US)
(74) Representative: Harding, Charles Thomas
(86) International application number: US9303965
(87) International publication number: WO9322312

(56) References cited:
- EP-A- 0 451 835
- EP-A- 0 451 836
- WO-A-86/05181
- WO-A-90/00172
- US-A- 4 725 687
- US-A- 5 077 404

## Description

### Field of the Invention

This invention relates to certain deazaaminopterin compounds useful for the treatment of inflammatory disease, such as rheumatoid arthritis, as well as a process for making such compounds and a method of using such compounds.

### Background of the Invention

DeGraw et al., U. S. Patent 4,369,319, issued January 19, 1983, disclose a class of 10-deazaaminopterin compounds having the structure of formula:

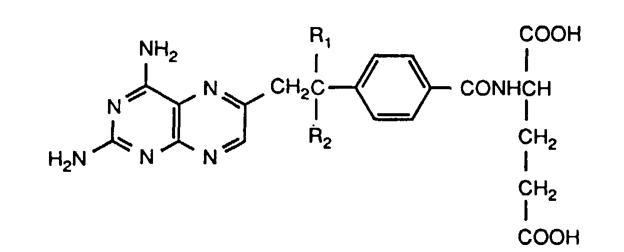

In the compound 10-deazaaminopterin, R₁ and R₂ are both hydrogen. In the alkyl derivatives of Patent No. 4,369,319, either or both of R₁ and R₂ is alkyl having from one to about eight, preferably one or two, carbon atoms. When only one of R₁ and R₂ is alkyl, the other is hydrogen. Exemplary R₁ and R₂ alkyl include methyl, ethyl, propyl, isopropyl, butyl, isobutyl, sec-butyl, tert-butyl, amyl, iso-amyl, sec-amyl, tert-amyl, hexyl, iso-hexyl, heptyl, iso-heptyl, octyl, iso-octyl, 2-ethyl hexyl and tert-octyl.

DeGraw et al., J.Med.Chem., 17, 552 (1974), report on the synthesis and antifolate activity of 10-deazaaminopterin. The antimicrobial and antitumor activities of the powerful dihydrofolic reductase inhibitors aminopterin and its N-10 methyl derivative, methotrexate, are well known, and numerous analogues have been made to further improve the potency, cell penetration, and toxicity properties of these compounds. As part of a continuing program to investigate structure-activity relationships in folic acid analogues, DeGraw et al. were interested in the effects of replacement of the nitrogen atom in the side chain of aminopterin and reported on the synthesis and biological activity of 10-deazaaminopterin. Continuing work with 10-deazaaminopterin and its 10-alkyl derivatives led to the discovery of their antileukemic activity, and to their efficacy in treating various ascites tumor systems.

In accordance with U. S. Patent 4,369,319, it was determined that leukemia, as well as other malignancies, including ascitic tumors, can be ameliorated in warm-blooded lower animals by the administration of 10-deazaaminopterin, a nontrivial analogue of methotrexate, the current drug of choice for the treatment of leukemia in the clinic, as well as 10-alkyl derivatives of 10-deazaaminopterin, and it is expected that these compounds will have a similar effect in humans.

Rheumatoid arthritis is an inflammation of the joints arising from infectious, metabolic, or constitutional causes, usually of unknown origin. It can result in serious restriction of movement and even invalidism. Since rheumatoid arthritis is a common disease that affects 2-3 million people in the United State alone, it poses a serious treatment problem. A substantial proportion of affected individuals will develop erosive joint disease and require surgical joint replacement despite therapies including disease-modifying antirheumatic drugs such as gold complexes, penicillamine, antimalarials, and methotrexate. In some patients with intractable rheumatoid arthritis, immunosuppressive agents including azathioprine, methotrexate, cyclophosphamide, and combinations of these drugs have proven beneficial. However, the potential side effects of some of these drugs, including bone marrow toxicity and neoplasia, have limited their frequency of use and the dose that is given.

The disease is one of a number of forms of proliferative disease, and the development of drugs for amelioration or curing the disease has occupied the attention of research organizations for many years, until most recently without appreciable success.

The antifolic acid drug methotrexate has been used as an antitumor agent since 1955. Its cytotoxic action in tumors is related to its ability to inhibit (essentially irreversibly) the key enzyme, dihydrofolate reductase, required for biosynthesis of tetrahydrofolic acid. Tetrahydrofolate is a vital component in one-carbon metabolism in cells, being required for biosynthesis of purine and pyrimidine nucleosides of the DNA and RNA. The drug is a powerful cytotoxic agent whose principal toxicities occur with liver, kidney, and mucosal tissue. Liver toxicity is the paramount concern for use in chronic therapy in a disease such as arthritis.

The ability of methotrexate to affect the inflammatory conditions of rheumatoid arthritis may be linked to its cytotoxic behavior. This may be in the nature of immune suppression and could involve attack on inflammatory phagocytic cells such as macrophages or neutrophils and T-helper cells in the synovial region. Very few methotrexate analogs have been evaluated against arthritis in animals, and there is no clear indication whether the antiarthritic properties are directly proportional to cytotoxicity. Galivan et al., Chem. Biol. Pteridines, DeGuyter, Berlin, 847 (1986), showed that adjuvant arthritis and streptoccocal cell wall arthritis in rats responded to doses of methotrexate relative to those used in man for treatment of rheumatoid arthritis. They also found that timing of dosage was most important for reduction of inflammation. Both methotrexate and aminopterin were found to inhibit inflammation, but other antifolate compounds that did not possess a 2,4-diaminopyrimidine unit or a benzoylglutamate side chain were ineffective.

Piper et al., J. Med, Chem., 25, 877-880 (1982), prepared the N-10 propyl, octyl, and propargyl analogues of methotrexate for evaluation. Biological evaluations of the three compounds consisted of studies of their effects on enzyme inhibition [(dihydrofolate reductase (EC 1.5.1.3) and thymidylate synthase)], L1210 cell growth inhibition, cellular membrane transport with various murine cell types (L1210, S180, Ehrlich, and epithelial), *in vivo* (mice) activity vs. L1210 leukemia and S180 ascites, and plasma clearance in mice. The *in vivo* results versus S180 ascites offered evidence that the propargyl compound might have a better therapeutic index against this tumor than methotrexate, but no other result from either of these compounds suggested significant superiority over methotrexate.

Taylor et al., J. Org. Chem., 48, 4852-4860 (1983) report that L-5-deazaaminopterin is equipotent with methotrexate both as an inhibitor of bovine liver dihydrofolate reductase and of L1210 murine leukemia cells. It is also equipotent with methotrexate *in vivo* both against L1210 and P388 leukemia in BDF₁ mice.

Piper, J. Med. Chem., 29, 1080-1087 (1986) report evidence indicating that modifications at the 5-and 10-positions of classical folic acid antimetabolites lead to compounds with favorable differential membrane transport in tumor versus normal proliferative tissue. The 5-alkyl-5-deaza analogues were also investigated, including 5-methyl-5-deazaaminopterin, 5-methyl-5-deazamethotrexate, and 5-methyl-10-ethyl-5-deazaaminopterin. Biological evaluation of the 5-methyl-5-deaza analogues, together with previously reported 5-deazaaminopterin and 5-deazamethotrexate, for inhibition of dihydrofolate reductase (DHFR) isolated from L1210 cells and for their effect on cell growth inhibition, transport characteristics, and net accumulation of polyglutamate forms in L1210 cells revealed the analogues to have essentially the same properties as the appropriate parent compound, aminopterin or methotrexate, except that the last two were approximately 10 times more growth inhibitory than methotrexate. In *in vivo* tests against P388/O and P388/methotrexate leukemia in mice, the analogues showed activity comparable to that of methotrexate, with the more potent 20 producing the same response in the P388/O test as methotrexate but at one-fourth the dose; none showed activity against P388/methotrexate.

DeGraw et al., J. Heterocyclic Chem., 88, 1 (1986), describe the synthesis of 5,10-dideazaaminopterin by two independent routes. Condensation of the piperidine enamine of 4-*p*-carbomethoxyphenylbutyraldehyde with ethoxymethylenemalononitrile followed by treatment of the resultant arylethylenaminomalononitrile with methanolic ammonia produced 2-amino-3-cyano-5-*p*-carbomethoxyphenethylpyridine. Cyclization of the aminocyanopyridine with guanidine afforded 4-amino-4-deoxy-5,10-dideazapteroic acid. Coupling of the pteroate intermediate with glutamate yielded the target 5,10-dideazaaminopterin. Alternatively, reduction of 2,4-diamino-6-formyl-5-deazapteridine with sodium borohydride gave the 6-hydroxymethyl compound. Conversion to the bromide was followed by alkylation of dimethyl homoterephthalate to afford methyl 4-amino-4-deoxy-10-carbomethoxy-5,10-dideazapteroate. Decarboxylation with ester cleavage (sodium cyanide in dimethyl sulfoxide at 180°) also gave the diaminopteroic acid. They reported that 5,10-dideazaaminopterin was an effective growth inhibitor of folate dependent bacteria, *S. faecium* and *L. casei.*

Sirotnak et al, Cancer Research. 5686-5691 (1988), describe studies examining a new class of 4-aminofolate analogues modified by an N to C conversion and alkyl substitution at the N-5 position of aminopterin and methotrexate. All of these analogues were equivalent to aminopterin and methotrexate as inhibitors of tumor cell dihydrofolate reductase (K₁ = 3.49-5.16 pM).

Piper et al., Chemistry and Biology of Pteridines, 1989, Walter de Gruyter & Co., Berlin - New York, state that modifications at the 5- and 10-positions of the classical antifolate structure have produced agents with antitumor activity superior to that of methotrexate. Examples are found in 5-alkyl-5-deaza analogues of aminopterin and methotrexate and in the 10-deazaaminopterin series, particularly 10-ethyl-10-deazaaminopterin whose ongoing clinical trials have produced highly favorable results). The 5-alkyl (methyl or ethyl) derivatives of 5-deazaaminopterin and 5-deazamethotrexate are equivalent to the parent compounds and to aminopterin and methotrexate as inhibitors of tumor cell dihydrofolate reductase, and overall, the activity of the 5-alkyl-5-deazamethotrexate derivatives appears to be equivalent to that of the 10-alkyl-10-deazaaminopterin types. In continuing studies on the effects of changes at positions 5 and 10, they synthesized 10-ethyl-5-methyl-5,10-dideazaaminopterin, 10-propargyl-5-deazaaminopterin, and 10-propargyl-5-methyl-5-deazaaminopterin. The syntheses and available data from biological evaluations are reported.

DeGraw et al., J. Med. Chem., 83, 678 (1990), report the synthesis of the 10-methyl and 10-ethyl analogues of 5,10-dideazatetrahydrofolic acid (DDTHF), a potent inhibitor of glycinamide ribotide (GAR) formyltransferase. Key intermediates in the process were 10-methyl- and 10-ethyl-4-amino-4-deoxy-5,10-dideazapteroic acid. Condensation of the piperidine enamines of branched 4-(*p*-carbomethoxyphenyl)butyraldehydes with (acetoxymethylene)malo-nonitrile afforded 1,1-dicyano-4-piperidinobutadiene. Subsequent reaction with alcoholic ammonium hydroxide yielded the appropriately substituted 2-amino-3-cyanopyridines. Ring closure with guanidine gave 10-methyl- and 10-ethyl-4-amino-4-deoxy-5,10-dideazapteroic acids. Coupling with diethyl glutamate followed by ester hydrolysis afforded 10-alkyl-5,10-dideazaaminopterin analogues which were effective inhibitors of DHFR derived from L1210, but were less potent than methotrexate for inhibition of growth of L1210 in culture.

What is needed is an effective treatment for inflammatory disease, such as rheumatoid arthritis, which exhibits relatively low toxicity compared to current treatments.

According to one aspect ofthe present invention there is provided
5-deazaaminopterin and 5,10-dideazaaminopterin compounds represented by the formula I : 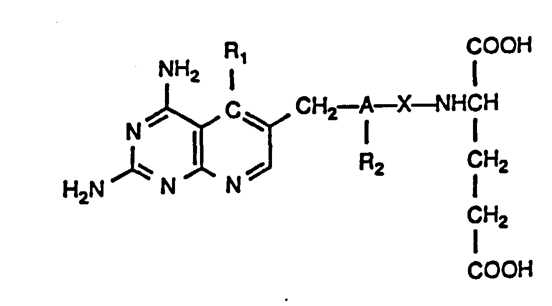 wherein
A is N;
X is one of 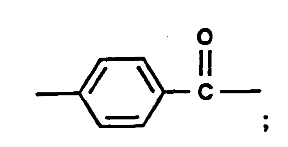 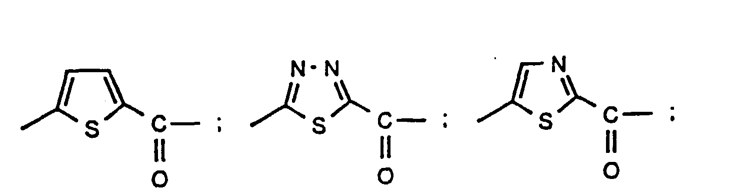 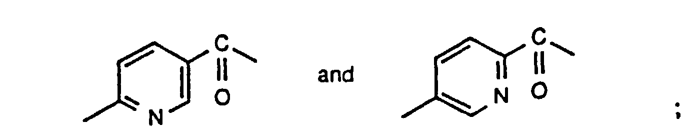 and R₁ and R₂ each represents hydrogen, or alkyl, alkenyl, or alkynyl group of up to eight carbon atoms;
provided that when X is 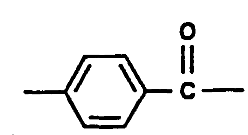 then R₁ is alkyl, alkenyl or alkynyl and when R₁ is alkyl, then R₂ is alkenyl or hydrogen provided R₁ is not methyl or ethyl when R₂ is hydrogen, and the pharmaceutically acceptable salts thereof.

Exemplary R₁ and R₂ alkyl include methyl, ethyl, propyl, iso-propyl, butyl, isobutyl, sec-butyl, tert-butyl, amyl, isoamyl, sec-amyl, tert-amyl, hexyl, iso-hexyl, heptyl, iso-heptyl, octyl, iso-octyl, 2-ethyl hexyl and tert-octyl.

Exemplary R₂ alkenyl include allyl, 1-propenyl, crotyl (2-butenyl), 2-pentenyl, 4-pentenyl, 2-hexenyl, 5-hexenyl, 3-isopropenyl, 3-isobutenyl, and 4-octenyl.

Exemplary R₂ alkynyl include propargyl, 2-butynyl, 3-butynyl, 4-pentynyl, 3-hexynyl, and 7 octynyl.

The invention also provides a medicament for treating rheumatoid arthritis and other proliferative diseases. Thus, according to another aspect ofthe present invention there is provided use of a 5-deazaaminopterin and a 5, 10-dideazaaminopterin of the formula I : 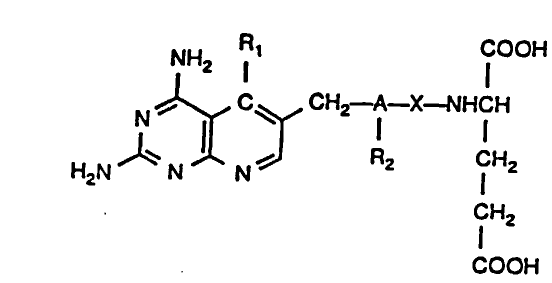 where
A is N;
X is one of 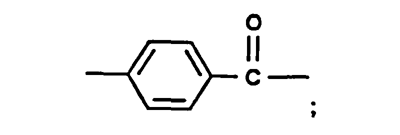 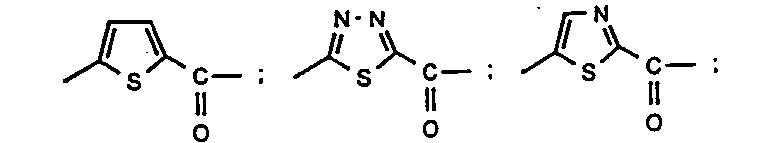 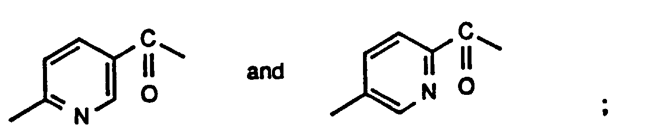 and R₁ and R₂ each represents hydrogen, or alkyl, alkenyl, or alkynyl group of up to eight carbon atoms; or a pharmaceutically acceptable salf thereof, for the manufacture of a medicament for use in the treatment of an inflammatory disease.

The salts may be formed with one or more free NH₂ groups and/or COOH groups of the 5-deazaaminopterin or 5, 10-dideazaaminopterin compound.

Those compounds in which X is 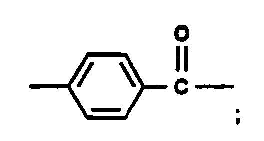 and R₁ and R₂ have from three to about eight carbon atoms, preferably from three to about five carbon atoms, are believed to be novel, and in addition, are exceptionally effective in the treatment of arthritis, in fact from six to ten times more effective than those compounds in which R₁ and R₂ have one or two carbon atoms, so as to constitute a delineated subclass within the larger genus. These compounds are therefore especially preferred.

This subclass of compounds within the invention accordingly is defined by the structure of Formula II : 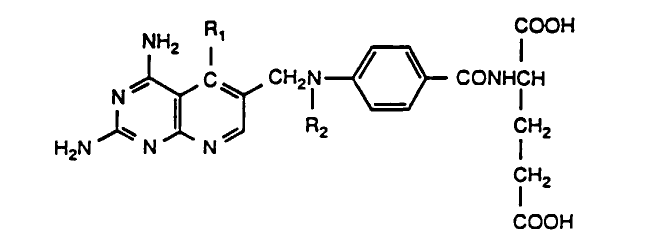 wherein R₁ and R₂ are alkyl, alkenyl, or alkynyl having from three to about eight, preferably from three to five, carbon atoms.

Exemplary 5-deazaaminopterin and 5, 10-dideazaaminopterin compounds falling within Formulae I or II are shown in the following Table IA.

**Table IA**

| **Compound No.** | **R**_{**1**} | **R**_{**2**} |
|---|---|---|
| 1 | H | H |
| 2 | H | CH₃ |
| 3 | H | C₂H₅ |
| 4 | H | C₃H₇ |
| 5 | H | CH₂=CHCH₂- |
| 6 | H- | CH≡CCH₂- |
| 7 | H | C₅H₁₁ |
| 8 | H | C₈H₁₇ |
| 9 | CH₃ | H |
| 10 | CH₃ | CH₃ |
| 11 | CH₃ | C₂H₅ |
| 12 | CH₃ | C₃H₇ |
| 13 | CH₃ | CH₂=CHCH₃ |
| 14 | CH₃ | CH≡CCH₂ |
| 15 | CH₃ | C₈H₁₇ |
| 16 | C₂H₅ | H |
| 17 | C₂H₅ | CH₃ |
| 18 | C₂H₅ | C₂H₅ |
| 19 | C₂H₅ | CH₂=CHCH₂ |
| 20 | C₂H₅ | CH≡CCH₂ |
| 21 | C₃H₇ | H |
| 22 | C₃H₇ | CH₃ |
| 23 | *i*-C₃H₇ | H |
| 24 | *i*-C₃H₇ | CH₃ |
| 25 | n-C₄H₉ | H |
| 26 | n-C₄H₉ | CH₃ |
| 27 | CH₂=CH-CH₂- | H |
| 28 | CH₂=CHCH₂ | CH₃ |
| 29 | CH≡CCH₂ | H |
| 30 | CH≡CCH₂ | CH₃ |
| 31 | C₅H₁₁ | H |
| 32 | C₈H₁₇ | H |

One subclass of thienyl compounds and thienyl analogues within the invention is defined by Formula IV: 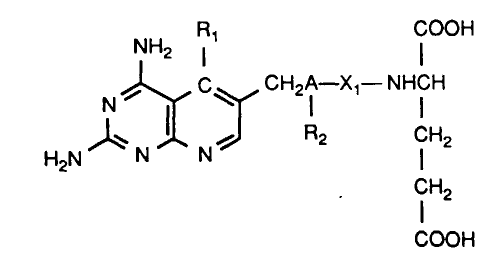 wherein
A is N
X₁ is one of 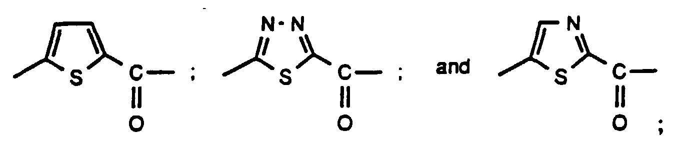
and R₁ is hydrogen or alkyl having from one to about eight, preferably from one to three, carbon atoms;
and R₂ is hydrogen or alkyl, alkenyl, or alkynyl having from one to about eight, preferably from one to three, carbon atoms.

Another subclass of pyridyl compounds within the invention is defined by Formula V: 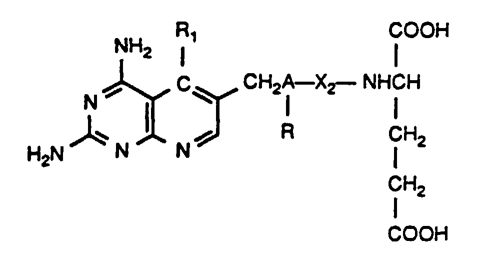 wherein
A is is N;
X₂ is one of 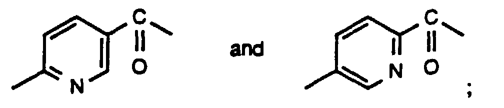
and R₁ is hydrogen or alkyl having from one to about eight, preferably from one to three, carbon atoms;
and R₂ is hydrogen or alkyl, alkenyl, or alkynyl having from one to about eight, preferably from one to three, carbon atoms.

Exemplary deazaaminopterin compounds falling within Formulae I, IV, and V are shown in the following Table IB.

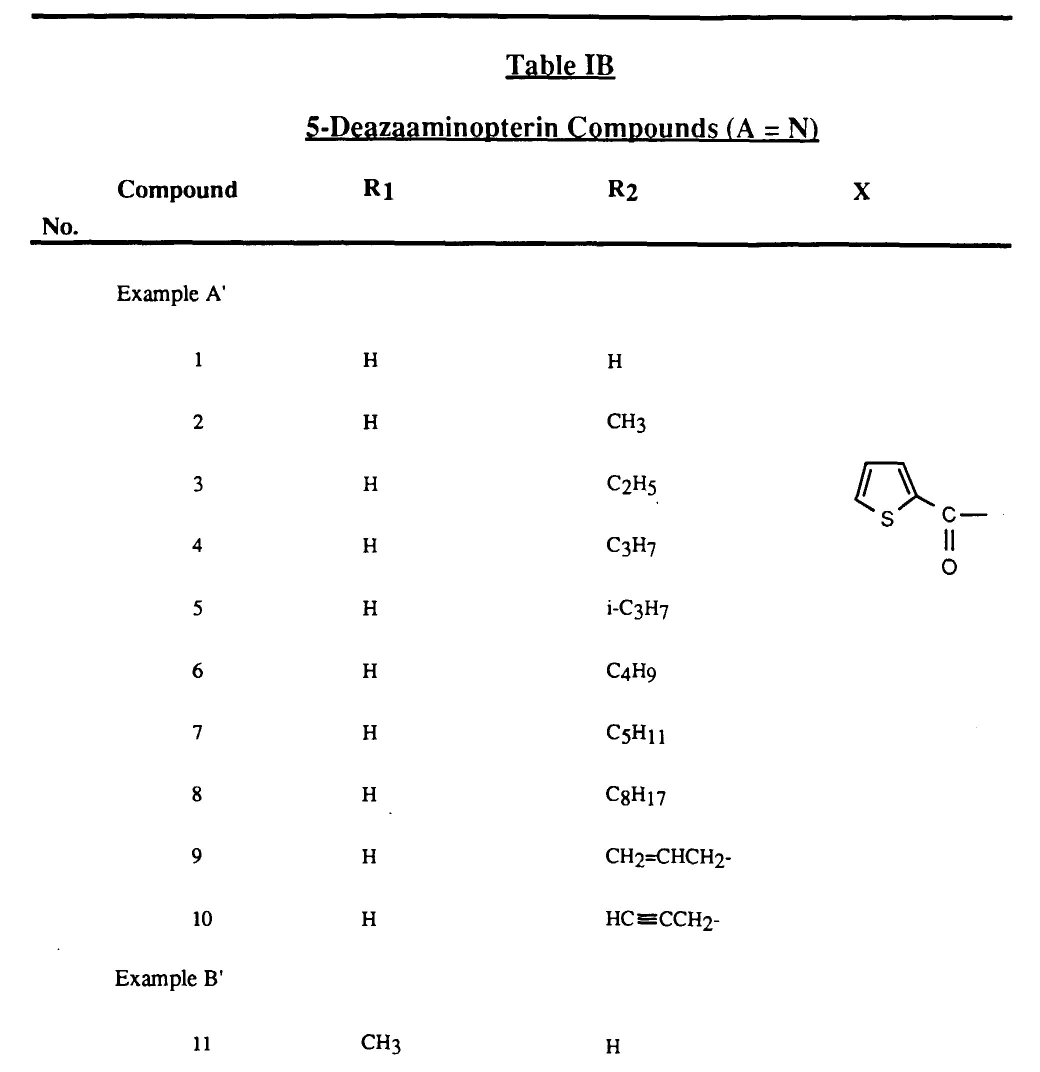

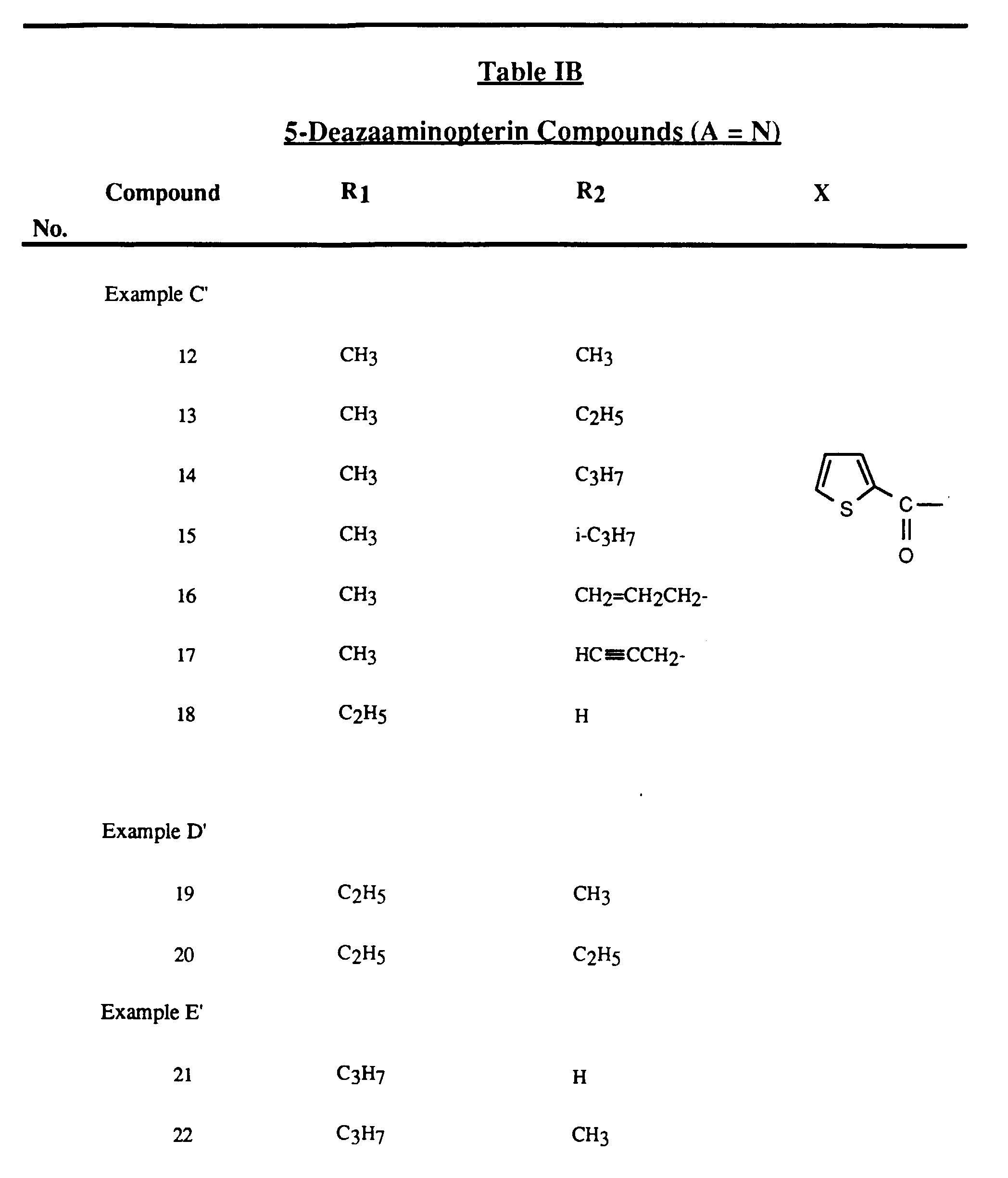

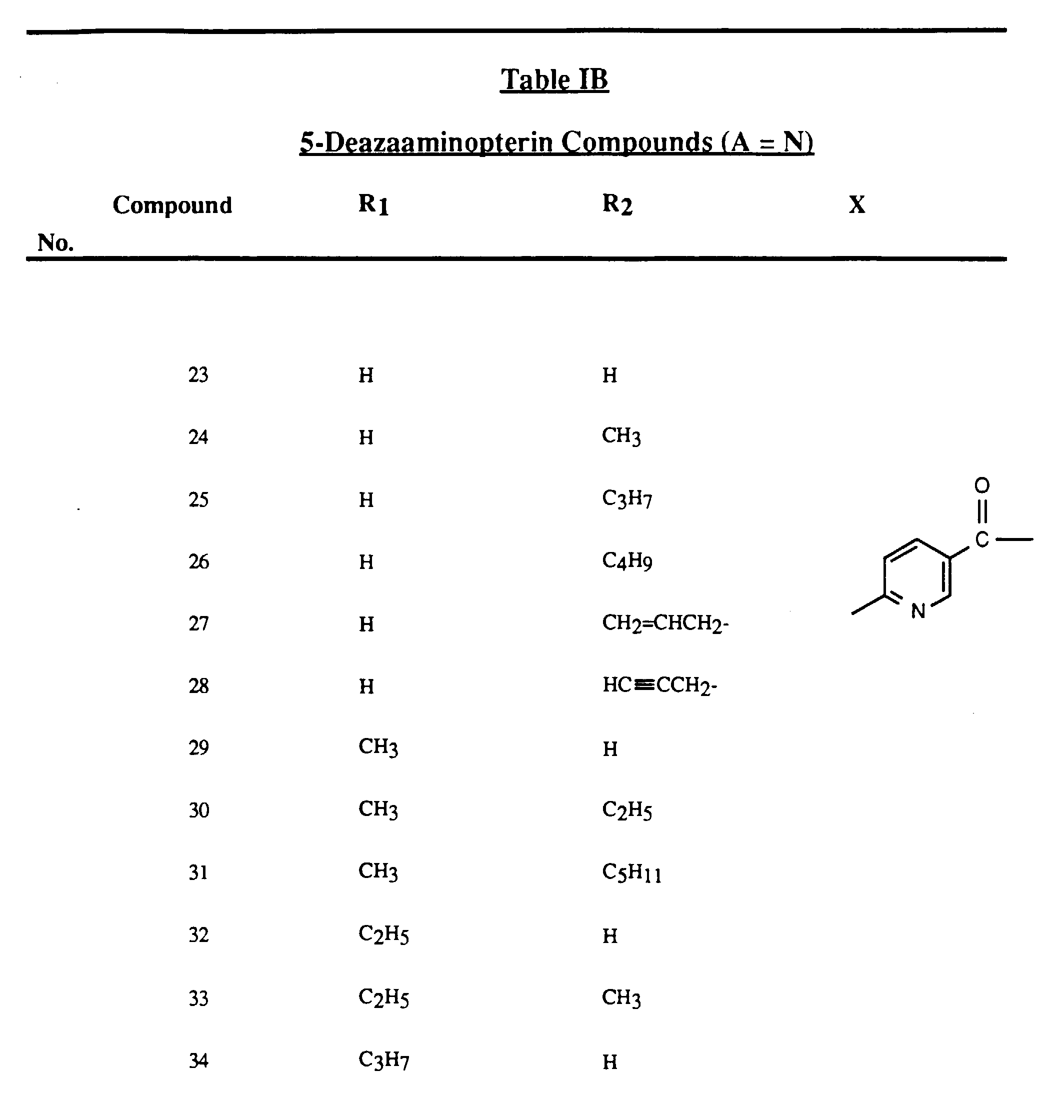

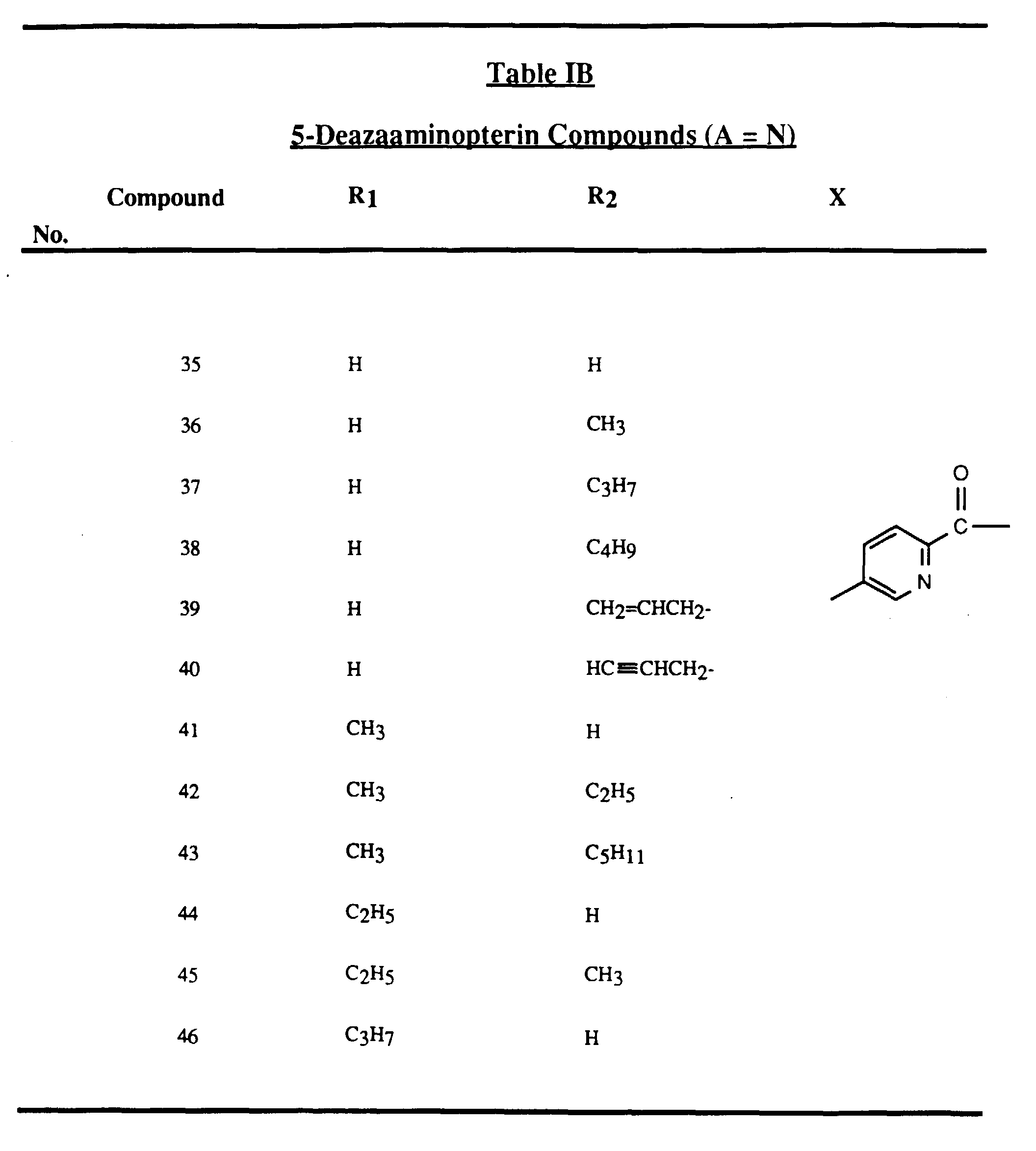

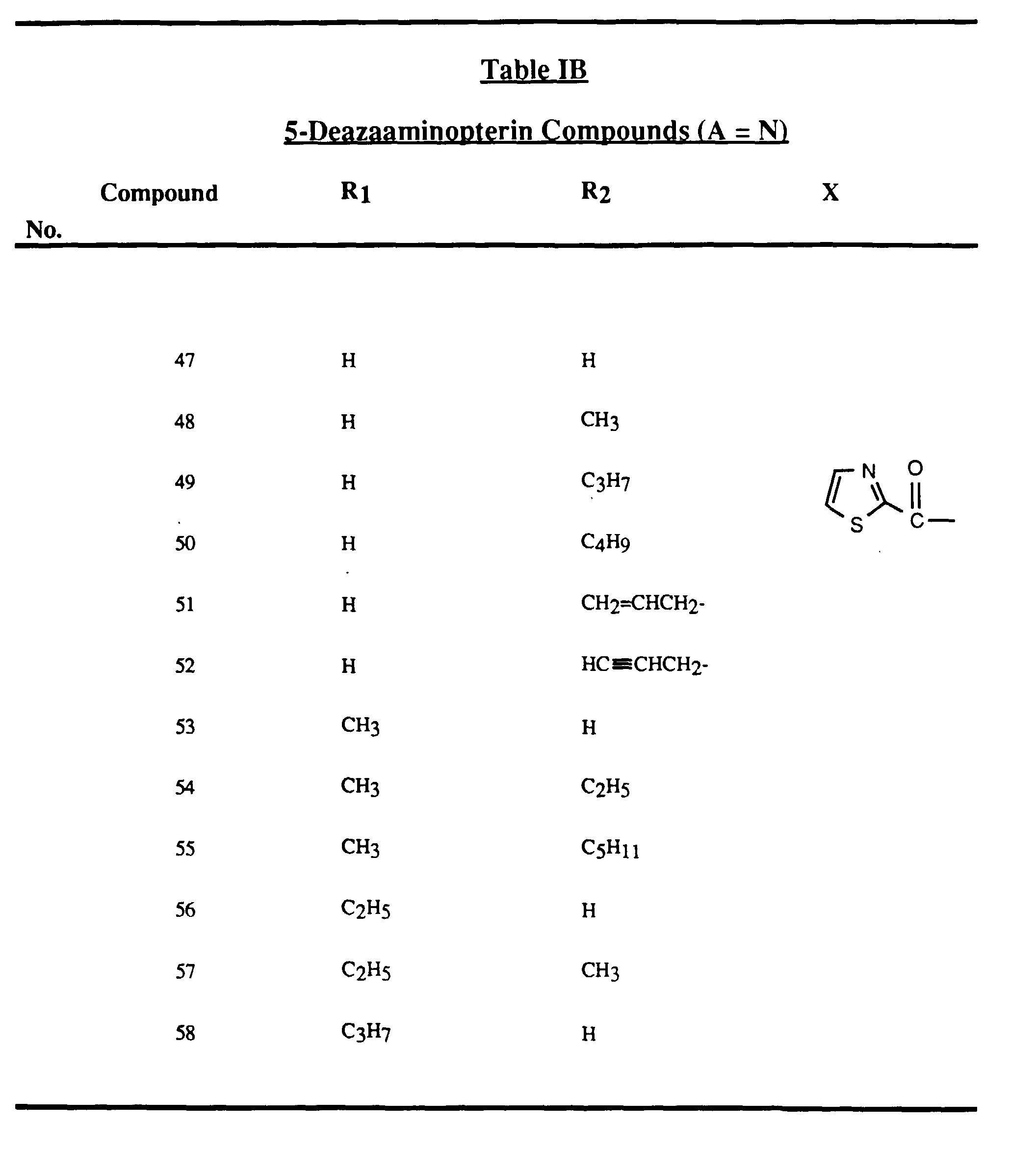

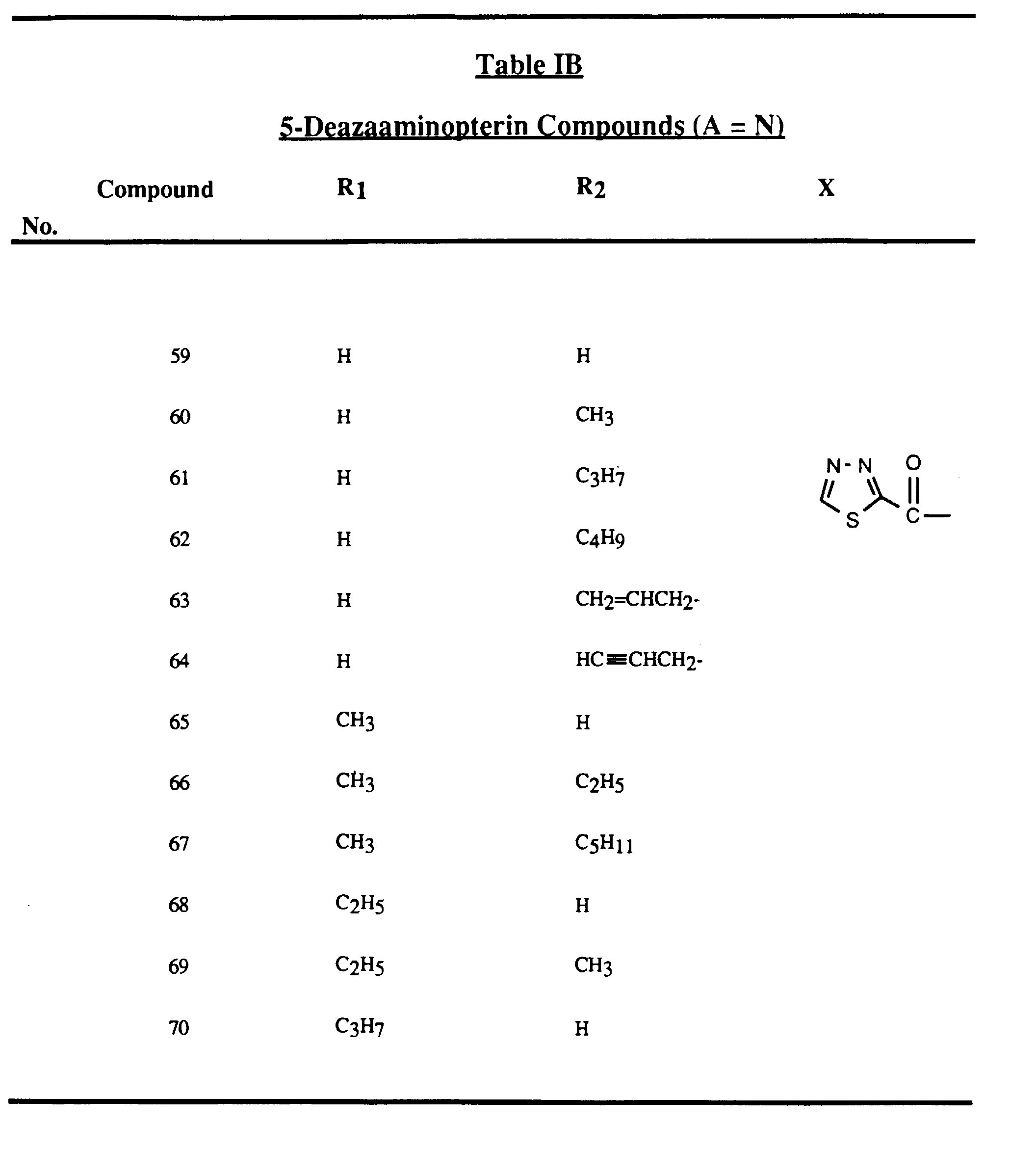

The synthesis of the compounds of Formula II, wherein A is N and X is 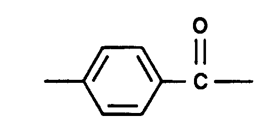 is adapted from that reported by Piper et al., J. Med. Chem., 29, 1080-1087 (1986), as summarized in Procedure I below.

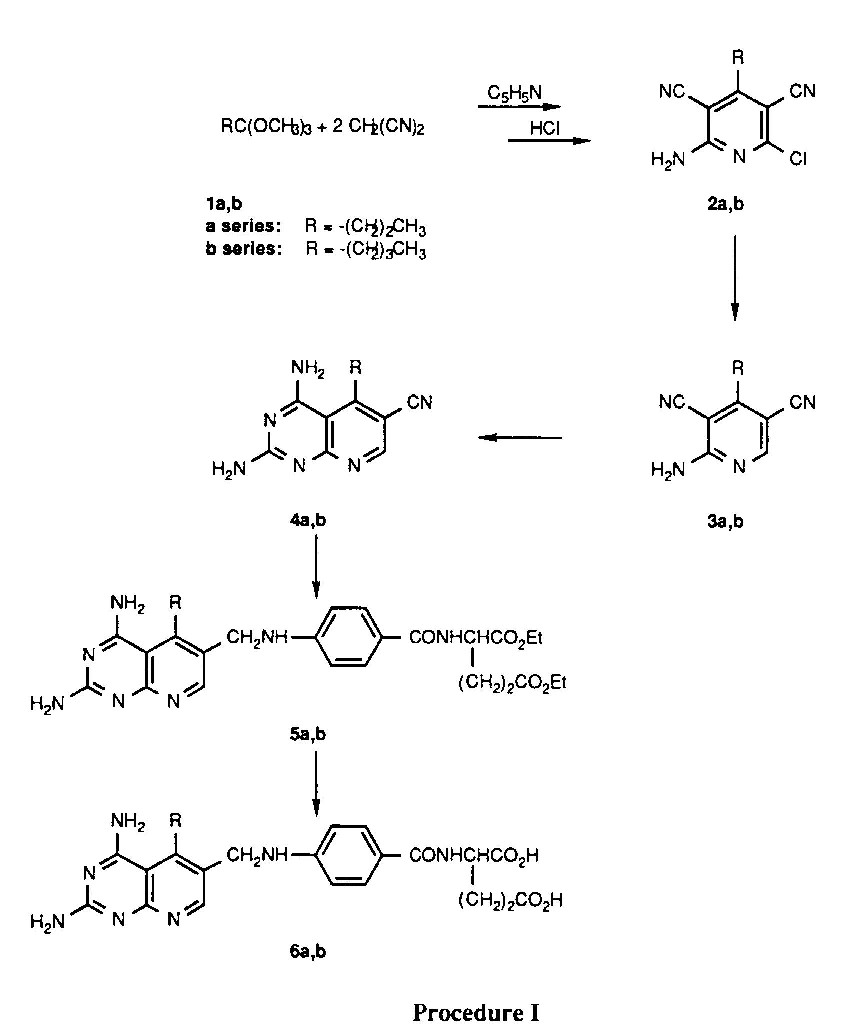

The synthesis of compounds of Formulae I, IV, and V wherein A is N and X is any of the heterocyclic rings set out for these formulae can be carried out by Procedure II, summarized hereinbelow.

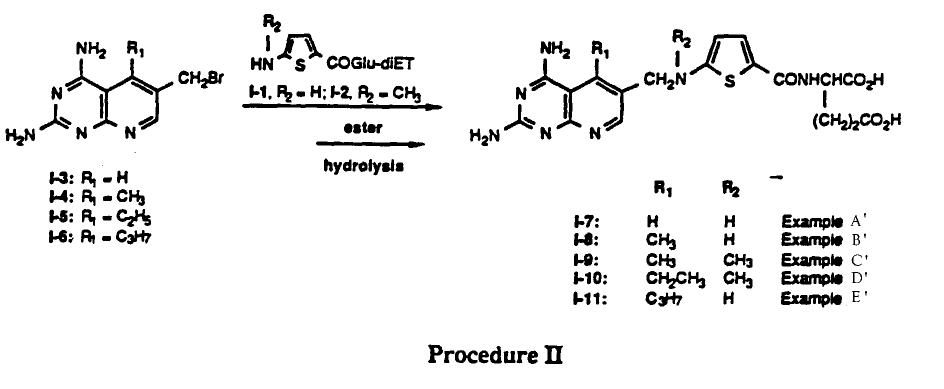

The following Examples A-B represent typical preparations of compounds represented by Formulae I and II using Procedure I. Each compound was prepared at least twice by the procedure given. Reference numbers within the examples refer to steps or compounds prepared by steps as indicated in Procedure I or compounds listed in Table 1A.

### Example A: Synthesis Of 5-Propyl-5-Deazaaminopterin (6a)

**2(a)** 2-Amino-6-chloro-4-propyl-3,5-pyridinedicarbonitrile. A solution of trimethylorthobutyrate (**1a**; 100 g, 0.670 mol), malononitrile (89.1 g, 1.35 mol), and pyridine (270 mL) was refluxed 1 hour. Excess pyridine was then removed by evaporation under reduced pressure (H₂O aspirator, bath to 60°C). The residue was treated with 12 *N* HCl (1.15 L) and the mixture was transferred to a 5-L three-necked flask equipped with a thermometer, condenser, and mechanical stirrer (Teflon paddle). The mixture was stirred rapidly while being heated at 85-90°C for 1 hour. Solid material formed during this time. The mixture was cooled to 20-25°C, and cold H₂O (3 L) was added. After the mixture had been kept in a refrigerator overnight, the solid was collected, washed thoroughly with H₂O and dried *in vacuo.* The product was homogeneous according to thin-layer chromatography (TLC) (EtOAc-cyclohexane, 1:1); yield 28% (42.4 g). Spectral data: mass, m/z 221, MH⁺ for C₁₀H₉ClN₄.

**3(a)** 2-Amino-4-propyl-3,5-pyridinedicarbonitrile. A solution of **2a** (42.3 g, 0.193 mol) in dimethylformamide (DMF) (600 mL) and triethyl amine (Et₃N) (70 mL) containing PdCl₂ (1.1 g) was shaken on a Parr apparatus under H₂ at 45 psi (310.3 kPa) for 16 hours. Examination by TLC revealed conversion was incomplete. The mixture was filtered from catalyst with the aid of a little DMF. Fresh PdCl₂ (1.1 g) and more Et₃N (35 mL) were added to the filtrate, and hydrogenation at 45 psi (310.3 kPa) was resumed. After 3 hours, TLC showed all **2a** had been converted. The mixture was filtered and the filtrate was concentrated under reduced pressure (< 1 mm, bath 30°C) to about 75-100 mL. Dilution with cold H₂O (1 L) caused precipitation of **3a**; yield 91% (32.6 g), homogeneous by TLC. Spectral data: mas, m/z 187, MH⁺ for C₁₀H₁₀N₄; ¹H NMR (Me₂SO-d₆) d 0.95 (t, 3, CH₃), 1.65 (m, 2, CH₂), 2.75 (t, 2, CH₂), 7.88 (br s, 2, NH₂), 8.52 (s, 1, C⁶-H).

**4(a)** 2,4-Diamino-5-propylpyrido[2,3-d]pyrimidine-6-carbonitrile. Anhydrous guanidine•HCl (6.15 g, 0.0640 mol) and NaOMe (3.49 g, 0.0650 mol) were combined in dry 2-(2-methoxyethoxy)ethanol (270 mL), and the mixture was stirred for about 0.5 hour before it was combined with a solution of **3a** (12.0 g, 0.0640 mol) in 2-(2-methoxyethoxy)ethanol (335 mL). The stirred mixture was heated under N₂ at 150-160°C for 7 hours. This mixture was allowed to cool to about 110°C while another solution of guanidine (one-half the previous amount) in 2-(2-methoxyethoxy)ethanol was prepared. The second guanidine solution was added, and heating at 150-160°C was resumed. After 5 hours, the mixture was allowed to cool, then evaporated *in vacuo* (<1 mm) to a viscous mixture. Addition of cold H₂O (∼500 mL) gave a crude solid, which was collected and dried *in vacuo.* The crude product mixture (11.4 g) was dissolved in DMF, and the solution was swirled with silica gel (about 40 g of 60-200 mesh). Evaporation *in vacuo* as before gave a solid dispersion of crude product mixture and silica gel. The dispersion was pulverized, dried further *in vacuo,* then applied to a column (9 ¥ 50-cm) of silica gel (60-200 mesh poured from CHCl₃). Gravity elution by CHCl₃-MeOH (95:5) followed, and fractions homogeneous in **4a** (R_{f} ∼0.55 on TLC using CHCl₃-MeOH, 5:1) were combined for evaporation to give pure **4a** (3.3 g, 23% yield). Spectral data: mass, m/z 229, MH⁺ for C₁₁H₁₂N₆; ¹H NMR (Me₂SO-d₆) d 0.92 (t, 3, CH₃), 1.62 (m, 2 CH₂ CH₂CH₃), 3.20 (m, 2, CH₂CH₂CH₃), 6.8-7.0 (br, 2, NH₂), 7.32 (br s, 2, NH₂), 8.78 (s, 1, C⁷-H).

**5(a)** Diethyl N-[4-[[(2,4-Diamino-5-propylpyrido[2,3-d]pyrimidin-6-yl)methyl] amino]-benzoyl]-L-glutamate. A stirred solution of **4a** (1.21 g, 5.30 mmol) and diethyl N-(4-aminobenzoyl)-L-glutamate (2.33 g, 7.23 mmol) in glacial AcOH (250 ml) containing damp Raney Ni (about 8 g) was kept under H₂ at atmospheric pressure for approximately 4 hours or until H₂ absorption from a gas buret had ceased near 240 ml. The catalyst was removed by filtration, and the filtrate was evaporated (H₂O aspirator, bath 30°C). The residue was dissolved in the minimum volume of EtOH (12-15 mL), and the stirred solution was gradually treated with 3% Na₂CO₃ solution to pH 7.8. The resulting crude product mixture was collected with the aid of cold H₂O, dried, and dispersed onto silica gel (60-200 mesh) as described above for precursor **4a**. The dispersion was applied to a silica gel column (5 ¥ 50-cm) poured from CHCl₃. Elution by gravity flow with CHCl₃-MeOH (95:5) followed. After TLC showed all diethyl N-(4-aminobenzoyl)-L-glutamate and minor contaminants more mobile than **5a** had been eluted, the system was switched to 85:15 CHCl₃-MeOH. Fractions homogeneous with respect to **5a** (R_{f} ∼0.5 using CHCl₃-MeOH, 3:1) were combined and evaporated to give pure **5a** in 16% yield (470 mg). Spectral data: Mass, m/z 538, MH⁺; ¹H NMR d 0.92 (t, 3, CH₃), 1.12-1.22 (2 t, 6, CH₃CH₂O overlapping), 1.54 (m, 2, CH₂CH₂CH₃), 1.98 and 2.06 (2 m, 2, CHCH₂CH₂, nonequivalent), 2.42 (t, 2, CH₂CH₂CO), 3.02 (t, 2, CH₂CH₂CH₃), 4.00-4.14 (br m, 4, CH₃CH₂O overlapping), 4.32 (d, 2, CH₂NH), 4.38 (m, 1, CONHCH), 6.35 (br s, 2, NH₂), 6.56 (t, 1, CH₂NH), 6.66 and 7.68 (2 d, 4, C₆H₄), 7.05 (s, 2, NH₂), 8.25 (d, 1, CONH), 8.52 (s, 1, C⁷-H). Anal. Calcd. for C₂₇H₃₅N₇O₅• 0.5 H₂O; C, 59.33; H, 6.64; N, 17.94. Found: C, 59.24, 59.55, H, 6.56, 6.49; N, 17.68, 17.71.

**6(a)** N-[4-[[(2,4-Diamino-5-propylpyrido[2,3-d]pyrimidin-6-yl)methyl]amino]benzoyl]-L-glutamic Acid (5-Propyl-5-deazaaminopterin). A solution of **5a** (400 mg, 0.732 mmol) in MeOH (800 mL) was treated with 1N NaOH (1.7 ml), and the solution was kept at 20-25°C for 64 hours. MeOH was removed by evaporation under reduced pressure (H₂O aspirator, bath 20-25°C). Solution did not occur when the residue was treated with H₂O (50 ml), indicating that unchanged **6a** was still present. The mixture was again made a clear solution by adding MeOH (300 ml) back to the aqueous suspension. The solution was left 48 hours longer at 20-25°C when high performance liquid chromatography (HPLC) [Piper et al., J. Med. Chem., 29, 1080 (1986)] indicated that virtually all **5a** had been converted to either **6a** disodium salt or a monosodium salt. MeOH was again removed, and the aqueous residue (adjusted to 50 ml) was treated with more 1N NaOH (0.8 ml). After 4-5 more days at 20-25°C, HPLC showed that conversion to **6a** was complete. The solution was clarified (Norit, Celite) and treated with 1N HCl to precipitate **6a** at pH 3.8 as a pale beige solid; yield, 69% (260 mg), hydrated as indicated below. Assay by HPLC showed the product to be homogeneous. Spectral data: Mass, m/z 482, MH⁺; UV, 1ₘₐₓ 228 nm (e 38,300), 299 (22,500) at pH 1; 225 nm (e 34,400), 284 (26,500) at pH 7; 225 nm (e 32,000), 284 (26,700) at pH 13; ¹H NMR (Me₂SO-d₆) d 0.92 (t, 3, CH₃), 1.56 (m, 2, CH₂CH₂CH₃), 1.96 and 2.02 (two m, 2, CHCH₂CH₂, nonequivalent), 2.32 (t, 2 CH₂CH₂CO), 3.4 (t, 2, CH₂CH₂CH₃), 4.30 (d, 2 CH₂NH), 4.32 (m, 1, CONHCH), 6.54 (t, 1, CH₂NH), 6.64 and 7.66 (2 d, 4, C₆H₄), 6.65 (br, 2 NH₂), 7.24 (br, 2, NH₂), 8.06 (d, 1, CONH), 8.52 (s, 1, C⁷-H). Anal. Calcd. for C₂₃H₂₇N₇O₅•2 H₂O: C, 53.38; H, 6.04; N, 18.94. Found: C, 53.72, 53.61; H, 5.86, 5.84; N, 18.74, 18.75.

### Example B: Synthesis Of 5-Butyl-5-Deazaaminopterin

**2(b)** 2-Amino-6-chloro-4-butyl-3,5-pyridinedicarbonitrile. A solution of trimethyl orthovalerate (**1b**; 20.1 g, 0.124 mol) and malononitrile (16.4 g, 0.248 mol) in pyridine (50 mL) was refluxed 45 min, cooled, and evaporated. The residue was stirred with 12N HCl (210 mL) at 85°C (bath temp) for 45 min to give **2b** as an insoluble solid. After dilution with H₂O (100 mL), the mixture was chilled and the solid collected to give **2b** in 27% yield (7.96 g); homogeneous by TLC (cyclohexane-EtOAc, 1:1). Spectral data: Mass, m/z 235, MH⁺ for C₁₁H₁₁ClN₄.

**3(b)** 2-Amino-4-butyl-3,5-pyridinedicarbonitrile. Hydrogenolysis of **2b** (7.32 g, 31.2 mmol) in DMF (88 ml) containing PdCl₂) and Et₃N (9 ml) was conducted in a Parr shaker at 40 psi for 16 hours. Examination by TLC revealed absence of **2b**. The catalyst was removed by filtration, and the filtrate was diluted with H₂O to cause precipitation of **3b**. The collected solid was reprecipitated from a Norit-treated and filtered (Celite) solution in DMF (120 ml) by adding H₂O; yield, 90% (5.6 g); homogeneous by TLC (cyclohexane-ETOAc, 1:1). Spectral data: Mass, m/z 201, MH⁺ for C₁₁H₁₂N₄; ¹H NMR (Me₂SO-d₆) d 0.92 (t, 3, CH₃), 1.38 (m, 2, CH₂CH₂CH₂CH₃), 1.58 (m, 2, CH₂CH₂CH₂CH₃), 2.75 (t, 2, CH₂CH₂CH₂CH₃), 7.88 (br, 2, NH₂), 8.52 (s, 1, C⁶-H).

**4(b)** 2,4-Diamino-5-butylpyrido[2,3-d]pyrimidine-6-carbonitrile. The annulation of **3b** with guanidine was conducted as described for the conversion of **3a** to **4a**. A typical yield of pure **4b** after column chromatographic purification (as described for **4a**) was 20%; homogeneous by TLC (CHCl₃-MeOH, 7:1). Spectral data: Mass, m/z 342, MH⁺ for C₁₂H₁₄N₆; ¹H NMR d 0.88 (t, 3, CH₃), 1.34 (m, 2, CH₂CH₂CH₂CH₃), 1.56 (m, 2, CH₂CH₂CH₂CH₃), 3.24 (t, 2, CH₂CH₂CH₂CH₃), 6.9 (br, 2, NH₂), 7.34 (br, 2, NH₂), 8.76 (s, 1, C⁷-H).

**5(b)** Diethyl N-[4-[[(2,4-Diamino-5-butylpyrido[2,3-d]pyrimidin-6-yl)methyl]amino]-benzoyl]-L-glutamate. Reductive condensation of **4b** with diethyl N-(4-aminobenzoyl)-L-glutamate was conducted as described for **5a**. Pure **5b** was also isolated as described for **5a**. A typical yield of pure **5b** was 15%. Spectral data: Mass, m/z 552, MH⁺ for C₂₈H₃₇N₇O₅; ¹H NMR (Me₂SO-d₆) d 0.85 (t, 3, CH₃), 1.12-1.22 (2 t, 6, CH₃CH₂O, overlapping), 1.35 (m, 2, CH₂CH₂CH₂CH₃), 1.52 (m, 2, CH₂CH₂CH₂CH₃), 1.98 and 2.05 (2 m, 2, CHCH₂CH₂, nonequivalent), 2.42 (t, 2, CH₂CH₂CO), 3.04 (t, 2, CH₂CH₂CH₂CH₃), 4.0-4.15 (br m, 4, CH₃CH₂O, overlapping), 4.30 (d, 2, CH₂NH), 4.38 (m, 1, CONHCH), 6.24 (br s, 2, NH₂), 6.52 (t, 1, CH₂NH), 6.66 and 7.68 (2 d, 4, C₆H₄), 6.92 (br s, 2, NH₂), 8.24 (d, 1, CONH), 5.40 (s, 1, C⁷-H).

**6(b)** N-[[(2,4-Diamino-5-butylpyrido[2,3-d]pyrimidin-6-yl)methyl]amino]benzoyl]-L-glutamic Acid (5-butyl-5-deazaaminopterin. A solution of **5b** (50 mg, 0.091 mmol) in MeOH (150 ml) treated with 1N NaOH (0.24 ml) was kept at 20-25°C for 4 days. MeOH was evaporated *in vacuo* (H₂O aspirator, bath 25°C), and the residue was dissolved in H₂O (3 mL). After 30 hours at 20-25°C, the solution was carefully treated with 1N HCl to pH 3.8, where **6b** precipitated; yield, 51% (24 mg). Assay by HPLC showed the product to be of 99.4% purity; spectral data: Mass, m/z 496, MH⁺; UV, 1ₘₐₓ 300 nm (e 23,900) at pH 1; 287 nm (e 25,900) at pH 7; 287 nm (e 26,100) at pH 13; ¹H NMR (Me₂SO-d6) 0.85 (t, 3, CH₃), 1.35 (m, 2, CH₂CH₂CH₂CH₃), 1.52 (m, 2, CH₂CH₂CH₂CH₃), 1.96 and 2.02 (2 m, 2, CHCH₂CH₃, nonequivalent), 2.32 (t, 2, CH₂CH₂CO), 3.06 (t, 2, CH₂CH₂CH₂CH₃), 4.3 (m, 3, CH₂NH overlapping with CONHCH), 6.54 (t, 1, CH₂NH), 6.66 and 7.66 (2 d, 4, C₆H₄), 7.18 (br s, 2, NH₂), 8.08 (d, 1, CONH), 8.54 (s, 1 C⁷H). Anal. Calcd. for C₂₄H₂₉N₇O₅•1.5 H₂O: C, 55.17; H, 6.17; N, 18.76. Found: C, 55.12; H, 6.03; N, 18.59.

The Examples A'-E' which follow illustrate application of Procedure II, to the preparation of specific compounds of Formulae I, IV, and V, and represent preferred embodiments of the invention. Reference numbers within the examples refer to steps or compounds prepared by steps described in Procedure II, or compounds listed in Table 1B.

### Example A': Synthesis Of Compound 1, Table 1B By Procedure II

N-(5-Aminothiophene-2-carbonyl)-L-glutamic Acid Diethyl Ester (**I-1**). This compound was prepared by the method of Marsham et al., J. Med. Chem., 34, 1594 (1991).

6-(Bromomethyl)-2,4-diaminopyrido[2,3-d]pyrimidine (**I-3**). This intermediate was prepared from 2,4-diaminopyrido[2,3-d]pyrimidine-6-methanol by the procedure of Piper et al., J. Med. Chem., 35, 332 (1992). This particular preparation analyzed for a 1.75 HBr•0.25 CH₃COOH salt (formula wt. 406.7).

N-[5-[[(2,4-Diaminopyrido[2,3-d]pyridin-6-yl)methyl]amino]thiophene-2-carbonyl]-L-glutamic Acid (**I.7**). The bromomethyl compound **I-3** and the sidechain precursor **I-1** (1.2 mmol of each) were stirred with CaCO₃ (2.4 mmol) in Me₂NAc (15 mL) at 20-25°C for 4 days. The mixture was filtered, and the clear filtrate was added dropwise to excess 2.5% NaHCO₃ solution with stirring. The precipitate that formed was collected, dried, and chromatographed on silica gel with elution by CHCl₃-MeOH (2:1) to give essentially pure diethyl ester, mass spectrum, m/e 502 (MH+ for C₂₂H₂₇N₇O₅S); predominantly one peak (>92%) by HPLC; yield 91 mg (15%). For hydrolysis, the ester (90 mg) was stirred with 1 N NaOH (1.8 mL) for 5 h (solution occurred after 1.5 h). Acidification to pH 3.8 caused precipitation of the product; yield 67 mg (75%) mass spectrum, m/e 446, MH⁺ for C₁₈H₁₉N₇O₅S.

### Example B': Synthesis Of Compound 2, Table 1B By Procedure II

6-(Bromomethyl)-2,4-diamino-5-methylpyrido[2,3-d]pyrimidine (**I-4**). 2,4-Diamino-5-methylpyrido[2,3-d]pyrinfidine-6-methanol, [Piper et al., J. Med. Chem., 35, 3002 (1992)] (4.5 g, 22.0 mmol) was dissolved in glacial AcOH (200 mL) at 95°C. The solution was cooled to 25°C, then treated with stirring with 30% dry HBr in AcOH (400 mL). When addition was complete, a clear solution remained. The flask was stoppered securely, and the solution was kept at 20-25°C before it was added to Et₂O (2.2 L) with stirring. The precipitate that formed was collected under N₂, washed with Et₂O, and dried in vacua (P₂O₅ and NaOH pellets); yield 9.5 g of **I-4** hydrobromide solvated by AcOH; yield 99% (based on formulation shown below), mass spectrum, m/e 268 and 270, MH⁺ for C₉H₁₀BrN₅; 1H NMR (Me₂SO-d6) d 2.78 (s, 3, CH₃), 4.93 (s, 2, CH₂Br), 8.17 (s, 2, NH₂), 8.75 (s, 1, C₇H), 9.32 (s, 2, NH₂); solvation by CH₃CO₂H evidenced by methyl-group singlet at d 1.90 whose integral height is one-half that of the CH₃ group of **I-4**. Thus the molar ratio of **I-4** to CH₃CO₂H is 1:0.5. Anal. Calcd for C₉H₁₀BrN₅•1.7HBr•0.5CH₃CO₂H (formula wt. 435.7): C, 27.57; H, 3.17; N, 16.07. Found: C, 27.53, H, 3.37; N, 16.11.

N-[5-[[(2,4-Diamino-5-methylpyrido[2,3-d]pyrimidin-6-yl)methyl]amino]thiophene-2-carbonyl]-L-glutamic Acid (**I-8**). Bromomethyl compound **I-4** (3.5 g, 8.0 mmol), sidechain precursor **I-1** (2.7 g, 8.2 mmol), and CaCO₃ (1.63 g, 16.3 mmol) were combined in Me2NAc (50 mL). The stirred mixture was warmed briefly (5-10 min.) at 70°C. Reactants other than CaCO₃ dissolved readily. The mixture was stirred at 20-25°C in a stoppered flask under N₂ for 7 days. Inorganic matter was filtered off, and silica gel (25 g) was added to the filtrate. The slurry was evaporated in vacua, and the dispersion was pulverized for application on a silica gel column (4 x 50 cm). Elution by CHCl₃-MeOH (95 : 5) removed front-running impurities. The product was eluted with CHCl₃-MeOH (85 : 15). Appropriate fractions were combined and evaporated to give the diethyl ester of 1-8 (3.4 g, 81% yield). Mass spectrum, m/e 516, MH⁺ for C₂₃H₂₉N₇O₅S. 1-8 diethyl ester (258 mg, 0.50 mmol) was stirred 3 hours under N₂ with 1N NaOH (5.0 mL). The resulting clear solution was treated with 2N HCl to pH 3.5 to give **I-8**; yield 46% (0.11 g). Mass spectrum, 460, MH+; ¹H NMR (Me₂SO-d6) d 1.88, 1.98 (two m, 2, CHCH₂, nonequivalent), 2.30 (t, 2, CH₂CO), 2.66 (s, 3, CH₃), 4.25 (m, 1, CHCH₂), 5.94 (d, 1, 4-ArH adjacent to 5-ArNH), 6.68 (s, 2, NH₂), 7.2-7.35 (m, 3, NH₂ overlapping 5-ArNH), 7.48 (d, 1, 3-ArH), 7.98 (d, 1, CONH), 8.52 (s, 1, C7-H). Anal. Calcd for C₁₉H₂₁N₇O₅S•2.8H₂O: C, 44.74; H, 5.26; N, 19.23. Found: C, 44.78; H, 5.18; N, 19.49.

### Example C': Synthesis Of Compound 12, Table IB, By Procedure II

N-[5-(Methylamino)thiophene-2-carbonyl]-L-glutamic Acid Diethyl Ester (**I-2**). A solution containing **I-1** (1.78 g, 5.42 mmol), (i-Pr)₂NEt (1.0 mL, 0.74 g, 5.7 mmol), and Me₂SO4 (0.59 mL, 0.79 g, 6.2 mmol) in N,N-dimethylformamide (DMF, 20 mL) was warmed at 60°C for 2 h, then left at 20-25°C for 42 hours. The solution was evaporated in vacua (1 mm, bath 25-30°C), and the residue was dissolved in EtOAc-cyclohexane (1:1 by volume) for application to a silica gel column. Elution by the same solvent system afforded fractions homogeneous by TLC in **I-2**. Fractions were combined and evaporated to afford 16% yield (287 mg) of **I-2** as an amber oil. Mass spectrum, m/e 343, MH+ for C₁₅H₂₂N₂O₅S.

N-[5-[[(2,4-Diamino-5-methylpyrido[2,3-d]pyrimidin-6-yl)methyl]methylamino]thiophene-2-carbonyl]-L-glutamic Acid (**I-9**). This compound was prepared from **I-4** (0.91 mmol) and sidechain precursor **I-2** (0.96 mmol) by essentially the same procedure as described above for the preparation of **I-8**. After filtration, the reaction solution was evaporated (<1 mm, bath to 40°C). The residue was dissolved in CHCl₃-MeOH (6 : 1) for application to a silica gel column. Elution by CHCl₃-MeOH (6 : 1) gave fractions homogeneous by TLC (CHCl₃-MeOH, 4 : 1; Rf ∼0.5) which were combined and evaporated to give the diethyl ester of **I-9**; yield 27% (132 mg), Mass spectrum, m/e 530, MH+ for C₂₄H₃₁N₇O₅S. This sample was hydrolyzed as described for **I-8** to give pure 1-9•3H₂O in 80% yield (109 mg), Mass spectrum, m/e 474, MH+; 1H NMR (Me₂SO-d6) d 1.90, 200. (two m, 2, CHCH₂ nonequivalent), 2.32 (t, 2, CH₂CO), 2.60 (s, 3, 5-CH₃), 2.87 (s, 3, CH₃N), 4.30 (m, 1, CHCH₂), 4.50 (s, 2, CH₂N), 6.05 (d, 1, 4-ArH adjacent to 5-ArN), 6.68 (s, 2, NH₂), 7.32 (s, 2, NH₂), 7.58 (d, 1, 3-ArH), 8.06 (d, 1, CONH), 8.35 (s, 1, C7H). Anal. Calcd for C₂₀H₂₃N₇O₅S•3H₂O: C, 45.53; H, 5.54; N, 18.59. Found: C, 45.60; H, 5.28; N, 18.36.

### Example D': Synthesis Of Compound 19, Table IB, By Procedure II

6-(Bromomethyl)-2,4-diamino-5-ethylpyrido[2,3-d]pyrimidine (**I-5**). 2,4-Diamino-5-ethylpyrido[2,3-d]pyrimidinc-6-methanol (2.80 g, 12.8 mmol), prepared as described for the 5-CH₃ homologue [Piper et al., J. Med. Chem., 35, 3002 (1992)], was converted to **I-5** as described above for **I.4**; yield 5.3 g, Mass spectrum, m/e 282 and 284, MH+ for C₁₀H₁₂BrN₅; 1H NMR (MeSO-d6) d 1.24 (t, 3, CH₃), 3.24 (q, 2, CH₂), 4.94 (s, 2, CH₂Br), 8.14 (s, 2, NH₂), 8.80 (s, 1, C7-H), 9.22 (s, 2, NH₂); solvation by CH₃CO₂H is evidenced by methyl-group singlet at d 1.92 whose integral height is one-third that of the CH₃ group of **I-5**. Based on the results, the formulation for the product would be **I-5**•1.4HBr•0.33CH₃CO₂H (formula wt. 416.7).

N-[5-[[(2A-Diamino-5-ethylpyrido[2,3d] -pyrimidin-6-yl)methyl]methylamino]thiophene-2-carbonyl]-L-glutamic Acid (**I-10**). Alkylation of **I-2** by **I-5** (0.84 mmol of each) was done as described under the preparations of **I-8** and **I-9**. Purification of the ester produced was done as described for **I-9** with CHCl₃-MeOH (7 : 1) used as eluant. The yied of pure diethyl ester of **I-10** was 27% (124 mg); mass spectrum, m/e 544, MH+ for C₂₅H₃₃N₇O₅S. This sample was hydrolyzed as described for **I-8** (or **I-9**) to give **I-10**•3H₂O in 82% yield (102 mg), Mass spectrum, m/e MH+; 1H NMR (Me₂SO-d6) d 1,16 (t, CH₃CH₂), 1.90, 2.00 (two m, 2, CHCH₂ nonequivalent), 2.30 (t, 2, CH₂CO), 2.86 (s, 3, CH₃N), 3.00 (q, 2, CH₃CH₂), 4.28 (m, CHCH₂), 4.52 (s, 2, CH₂N), 6.05 (d, 1, 4-ArH adjacent to 5-ArN), 6.60 (s, 2, NH₂), 7.22 (s, 2, NH₂), 7.61 (d, 1, 3-ArH), 8.06 (d, 1, CONH), 8.40 (s, 1, C₇H). Anal. Calcd for C₂₁H₂₅N₇O₅S•3H₂O: C, 46.57; H, 5.77; N, 18.10. Found: C, 46.55,; H, 5.52; N, 18.15.

### Example E': Synthesis Of Compound 21, Table IB, By Procedure II

Preparation of 6-Bromomethyl-2,4-diamino-5-propylpyrido(2,3-d)pyrimidine (**I-6**). 2-Amino-6-chloro-4-propyl-3,5-pyridinedicarbonitrile. A solution of trimethyl orthobutyrate (1a; 100 g, 0.670 mol), malononitrile (89.1 g, 1.35 mol), and pyridine (270 mL) was refluxed 1 hour. Excess pyridine was then removed by evaporation under reduced pressure (H₂O aspirator, bath to 60°C). The residue was treated with 12 N HCl (1.15 L) and the mixture was transferred to a 5-L three-necked flask equipped with a thermometer, condenser, and mechanical stirrer (Teflon paddle). The mixture was stirred rapidly while being heated at 85-90°C for 1 hour. Solid material formed during this time. The mixture was cooled to 20-25°C, and cold H₂O (3 L) was added. After the mixture had been kept in a refrigerator overnight, the solid was collected, washed thoroughly with H₂O and dried in vacua. The product was homogeneous according to TLC (EtOAc-cyclohexane, 1:1); yield 28% (42.4 g). Spectral data: mass, m/z 221, MH+ for C₁₀H₉ClN₄.

2-Amino-4-propyl-3,5-pyridinedicarbonitrile. A solution of **2a** (42.3 g, 0.193 mol) indimethylformamide (DMF, 600 mL) and triethyl amine (Et₃N, 70 mL) containing PdCl₂ (1.1 g) was shaken on a Parr apparatus under H₂ at 45 psi (310.3 kPa) for 16 hours. Examination by TLC revealed conversion was incomplete. The mixture was filtered from catalyst with the aid of a little dimethyl formamide (DMF). Fresh PdCl₂ (1.1 g) and more Et₃N (35 mL) were added to the filtrate, and hydrogenation at 45 psi (310.3 kPa) was resumed. After 3 hours, TLC showed all **2a** had been converted. The mixture was filtered and the filtrate was concentrated under reduced pressure (< 1 mm, bath 30°C) to about 75-100 mL. Dilution with cold H₂O (1 L) caused precipitation of **3a**; yield 91% (32.6 g), homogeneous by TLC. Spectral data: mas, m/z 187, MH+ for C₁₀H₁₀N₄; 1H NMR (Me₂SO-d6)d 0.95 (t, 3, CH₃), 1.65 (m, 2, CH₂), 2.75 (t, 2, CH₂), 7.88 (br s, 2, NH₂), 8.52 (s, 1, C₆-H).

2,4-Diamino-5-propylpyrido[2,3-d]pyrimidine-6-carbonitrile. Anhydrous guanidine•HCl (6.15 g, 0.0640 mol) and NaOMe (3.49 g, 0.0650 mol) were combined in dry 2-(2-methoxyethoxy)ethanol (270 mL), and the mixture was stirred for about 0.5 hour before it was combined with a solution of 3a (12.0 g, 0.0640 mol) in 2-(2-methoxyethoxy)ethanol (335 mL). The stirred mixture was heated under N₂ at 150-160°C for 7 hours. This mixture was allowed to cool to about 110°C while another solution of guanidine (one-half the previous amount) in 2-(2-methoxyethoxy)ethanol was prepared. The second guanidine solution was added, and heating at 150-160°C was resumed. After 5 hours, the mixture was allowed to cool, then evaporated in vacua (<1 mm) to a viscous mixture. Addition of cold H₂O (~500 mL) gave a crude solid, which was collected and dried in vacua. The crude product mixture (11.4 g) was dissolved in DMF, and the solution was swirled with silica gel (about 40 g of 60-200 mesh). Evaporation in vacuo as before gave a solid dispersion of crude product mixture and silica gel. The dispersion was pulverized, dried further in vacuo, then applied to a column (9 ¥ 50-cm) of silica gel (60-200 mesh poured from CHCl₃). Gravity elution by CHCl₃-MeOH (95:5) followed, and fractions homogeneous in **4a** (Rf ∼0.55 on TLC using CHCl₃-MeOH, 5:1) were combined for evaporation to give pure **4a** (3.3 g, 23% yield). Spectral data: mass, m/z 229, MH+ for C₁₁H₁₂N₆; 1H NMR (Me₂SO-d6) d 0.92 (t, 3, CH₃), 1.62 (m, 2 CH₂CH₂CH₃), 3.20 (m, 2, CH₂CH₂CH₃), 6.8-7.0 (br, 2, NH₂), 7.32 (br s, 2, NH₂), 8.78 (s, 1, C₇-H).

2,4-Diamino-5-propylpyrido[2,3-d]pyrimidine-6-carboxaldehyde. Raney Ni (1.2 g damp) was added with the aid of 95-97% HCO₂H (7 mL) to a solution of 2,4-diamino-5-propylpyrido[2,3-d]pyrimidine-6-carbonitrile (500 mg, 2.19 mmol) in 95-97% HCO₂H (5 mL). The stirred mixture was heated at 75-80°C for 1.5 h. Raney Ni was removed by filtration, and the filtrate was evaporated. The residue was dissolved in hot H₂O (20 mL), and the solution was filtered, then cooled, and treated with concentrated NH₄OH to pH 7 to cause solid to precipitate. The mixture was kept at 0-5°C overnight before the solid was collected and washed with cold H₂O. This crude material was applied to a silica gel column and eluted with CHCl₃-MeOH (7:1). Evaporation of the product fractions afforded the aldehyde; yield 8% (41 mg). Anal. Calcd. for C₁₁H₁₃N₅O•0.2H₂O: C, 56.26; H. 5.75; N, 29.82. Found: C, 56.26; H, 5.70. N, 29.72. MS, m/e 232, MH+; UV, 1max (e ¥ 10-3) in 0.1N HCl, 235 (22.9), 257 (19.5), 316 (9.15); pH 7 buffer, 234 (15.7), 265 (17.4), 318 (11.0), 346 (12.2); in 0.1N NaOH, 234 (17.5), 265 (16.6), 347 (12.8); 1H NMR (Me₂SO-d6), d 0.92 (t, 3, CH₃), 1.55 (sext, 2, CH₂CH₂CH₃), 3.46 (t, 2, CH₂CH₂CH₃), 6.84 (br s, 2, NH₂), 7.30 (s, 2, NH₂), 8.88 (s, 1, C₇-H), 11.0 (s, 1, CHO).

2,4-Diamino-5-propylpyridol[2,3-d]pyrimidine-6-methanol. The aldehyde (95 mg, 0.41 mmol) was stirred with MeOH (20 mL), and the near-solution was treated with 3 portions of NaBH₄ (17 mg total, 0.45 mmol) added at 15-min intervals. Complete solution occurred after the first addition of NaBH₄. The solution was left at 20-25°C for 1 h. The solution was treated with H₂O (1 mL), neutralized (to pH 7) with glacial AcOH, and evaporated to near dryness. Solid residue was stirred with a little cold H₂O (~1 mL), collected, and dried to give a first crop of 6-hydroxymethyl compound; yield 31% (30 mg); MS, m/e 234, MH+ for C₁₁H₁₅N₅O: 1H NMR (Me₂SO-d6), d 0.96 (t, 3, CH₃), 1.56 (sext, 2, CH₂CH₂CH₃), 3.06 (t, 2, CH₂CH₂CH₃), 4.52 (s, 2, CH₂OH), 6.22 (s, 2, NH₂), 6.90 (s, 2, NH₂), 8.48 (s, 1, C₇-H). The filtrate from this sample was evaporated to dryness, and the residue was extracted several times with boiling EtOAc to provide another crop of hydroxymethyl compound (80 mg), MS m/e 234.

6-(Bromomethyl)-2,4-diamino-5-propylpyrido[2,3-d]pyrimidine (**I-6**). The hydroxymethyl compound was treated with dry HBr in AcOH as reported for the methyl homolog (**I-4**). Addition of Et₂O caused precipitation of yellow solid which was collected with the aid of Et₂O and dried in vacua to afford the HBr salt of the product; MS m/e 296 and 298, MH+ for C₁₁H₁₄BrN₅, but with higher mass peaks present. The 1H NMR spectrum of the product mixture showed expected singlets due to the CH₂Br (4.92) and the C₇-H (8.78). Relative integral values suggested about 10 mole-percent of the product.

N-[5-[[(2,4-Diamino-5-propylpyrido[2,3-d]pyrimidin-6-yl)methyl]amino]thiophene-2-carbonyl]L-glutamic Acid (**I-11**). A mixture of the crude bromomethyl preparation described above (0.55 g), diethyl N-(5-aminothiophene-2-carbonyl)-L-glutamate (0.40 g, 1.2 mmol) and CaCO₃ (160 mg) in Me₂NAc (7 mL) was stirred at 20-25°C for 4 days. Insoluble material was removed by filtration and the filtrate was evaporated to dryness. The residue was chromatographed on silica gel with elution by CHCl₃-MeOH (5:1) to give fractions homogeneous in the desired product. Evaporation of the pooled fractions gave the diethyl ester as a pale-orange solid (20 mg); MS m/e 545, MH+ for C₂₅H₃₃N₇O₅S. For ester hydrolysis, this sample was dissolved in 1 N NaOH (0.37 mL), and the solution was kept at 20-23°C for 5 h. The solution was clarified (Celite mat), then acidified to pH 3.8-4.0 with 2 N HCl. After the mixture had been refrigerated, the solid was collected and dried; yield 6 mg. MS, m/e 488, MH+ for C₂₁H₂₅N₇O₅S.

The deazaaminopterin compound can be administered per se, or in association with a pharmaceutically acceptable diluent or carrier. The invention accordingly also provides a pharmaceutical composition in dosage unit form comprising from 0.1 to about 500 mg of the deazaaminopterin compound, per dosage unit, together with a pharmaceutically acceptable nontoxic inert carrier or diluent therefore.

The deazaaminopterin compound can be used as such, or in the form of an acid addition salt. These salts are formed with one or more free NH₂ groups of the deazaaminopterin molecule. Typically, the compounds are injected in the form of their sodium salts in aqueous solution. Other salts, e.g., K, Ca, NH₄, etc. could be used as prepared from the appropriate hydroxide or carbonates.

The acid addition salts are preferably the pharmaceutically acceptable, nontoxic addition salts with suitable acids, such as those with inorganic acids, for example, hydrochloric, hydrobromic, nitric, sulphuric, and phosphoric acids; and with organic acids, such as organic carboxylic acids, for example, glycolic, maleic, hydroxymaleic, malic, tartaric, citric, calicylic, o-acetyloxybenzoic, nicotinic, and isonicotinic acid; and organic sulphonic acids, for example, methanesulphonic, ethanesulphonic, 2-hydroxyethanesulphonic, toluene-p-sulphonic, and naphthalene-2-sulphonic acid.

An acid addition salt can be converted into the free compound according to known methods, for example, by treating it with a base, such as with a metal hydroxide or alkoxide, for example, an alkali metal or alkaline earth metal hydroxide, for example, lithium hydroxide, sodium hydroxide, potassium hydroxide or calcium hydroxide; with a metal carbonate, such as an alkali metal or an alkaline earth metal carbonate or hydrogen carbonate, for example, sodium, potassium or calcium carbonate or hydrogen carbonate, with ammonia; or with a hydroxyl ion exchange resin, or with any other suitable reagent.

An acid addition salt may also be converted into another acid addition salt according to known methods; for example, a salt with an inorganic acid may be treated with a metal salt, for example a sodium, barium or silver salt, of an acid in a suitable diluent, in which a resulting inorganic salt is insoluble and is thus removed from the reaction medium. An acid-addition salt may also be converted into another acid addition salt by treatment with an anion exchange preparation.

The glutamic acid COOH groups can also be in salt form, as the ammonium NH₄, alkali metal salts (Na⁺⁺, K⁺), or the nontoxic alkaline earth metal salts (Ca⁺⁺) of the glutamate COOH groups.

The deazaaminopterin compound or salt thereof can be administered to the animal by any available route, including oral and parenteral (intravenous, intraperitoneal, subcutaneous, and intramuscular) administration. The amount administered is sufficient to ameliorate the arthritis or other proliferative disease, and will depend upon the type of arthritis, the species of animal, and the weight of the animal. For example, in human administration, a dosage of deazaaminopterin compound within the range from about 0.1 mg/kg to about 500 mg/kg per day should be sufficient. Dosages in the higher part of the range, approaching 500 mg/kg, are normally administered in conjunction with leucovorin (dl-r-formyl tetrahydrofolate) to reduce toxicity. In the treatment of lower test animals, a similar dosage range is therapeutic. The upper limit of dosage is that imposed by toxic side effects, and can be determined by trial and error for the animal to be treated, including humans.

To facilitate administration, the deazaaminopterin compound or salt thereof can be provided in composition form, and preferably in dosage unit form. While the compound can be administered per se, it is normally administered in conjunction with a pharmaceutically acceptable carrier therefor, which dilutes the compound and facilitates handling. The term "pharmaceutically acceptable" means that the carrier (as well as the resulting composition) is sterile and nontoxic.

The carrier or diluent can be solid, semisolid, or liquid, and can serve as a vehicle, excipient, or medium for the compound. Exemplary diluents and carriers include lactose, dextrose, sucrose, sorbitol, mannitol, starches, gum acacia, calcium phosphate, mineral oil, cocoa butter, oil of theobroma, alginates, tragacanth, gelatin, syrup, methyl cellulose, polyoxyethylene sorbitan monolaurate, methyl- and propylhydroxybenzoate, talc, or magnesium stearate.

For convenience in handling, the deazaaminopterin compound and carrier or diluent can be enclosed or encapsulated in a capsule, sachet, cachet, gelatin, paper, or other container, especially when intended for use in dosage units. The dosage units can for example take the form of tablets, capsules, suppositories, or cachets.

The following Examples 1-7 illustrate various forms of dosage units in which the deazaaminopterin compounds or salts thereof can be prepared:

### Example 1

| Tablet Formation | Mg/tablet |
|---|---|
| Deazaaminopterin compound | 1.5 |
| Lactose | 86 |
| Corn starch (dried) | 45.5 |
| Gelatin | 2.5 |
| Magnesium stearate | 1.0 |

The deazaaminopterin compound is powdered and passed through a mesh sieve and well mixed with the lactose and 30 mg of the corn starch, both passed through a sieve.

The mixed powders are massed with a warm gelatin solution, prepared by stirring the gelatin in water and heating to form a 10% w/w solution. The mass is granulated by passing through a sieve, and the moist granules dried at 40°C.

The dried granules are regranulated by passing through a seive and the balance of the starch and the magnesium stearate is added and thoroughly mixed.

The granules are compressed to produce tablets each weighing 150 mg.

### Example 2

| Tablet Formation | Mg/tablet |
|---|---|
| Deazaaminopterin compound | 100 |
| Lactose | 39 |
| Corn starch (dried) | 80 |
| Gelatin | 4.0 |
| Magnesium stearate | 2.0 |

The method of preparation is identical with that of Example 1, except that 60 mg of starch is used in the granulation process and 20 mg during tableting.

### Example 3

| Capsule formation | Mg/capsule |
|---|---|
| Deazaaminopterin compound | 250 |
| Lactose | 150 |

The deazaaminopterin compound and lactose are passed through a sieve and the powders well mixed together before filling into hard gelatin capsules of suitable size, so that each capsule contains 400 mg of mixed powders.

### Example 4

| Suppositories | Mg/suppositories |
|---|---|
| Deazaaminopterin compound | 50 |
| Oil of Theobroma | 950 |

The deazaaminopterin compound is powdered and passed through a sieve and triturated with molten oil of theobroma at 45°C to form a smooth suspension.

The mixture is well stirred and poured into molds, each of nominal 1 g capacity, to produce suppositories.

### Example 5

| Cachets | Mg/cachet |
|---|---|
| Deazaaminopterin compound | 100 |
| Lactose | 400 |

The deazaaminopterin compound is passed through a mesh sieve, mixed with lactose previously sieved and fitted into cachets of suitable size so that each contains 500 mg.

### Example 6

| Intramuscular injection (sterile suspension in aqueous vehicle) | Mg |
|---|---|
| Deazaaminopterin compound | 10 |
| Sodium citrate | 5.7 |
| Sodium carboxymethylcellulose (low viscosity grade) | 2.0 |
| Methyl para-hydroxybenzoate | 1.5 |
| Propyl para-hydroxylbenzoate | 0.2 |
| Water for injection to 1.0 ml | |

### Example 7

| Intraperitoneal intravenous or subcutaneous injection (sterile solution in aqueous carrier system) | Mg |
|---|---|
| Deazaaminopterin compound, hydrochloric acid addition salt | 15 |
| Sodium citrate | 5.7 |
| Sodium carboxymethylcellulose (low viscosity grade) | 2.0 |
| Methyl para-hydroxybenzoate | 1.5 |
| Propyl para-hydroxylbenzoate | 0.2 |
| Water for injection to 1.0 ml | |

### Example 8: In Vivo Biology Of Type II Collagen Arthritis And Methotrexate Treatment Using 5-Deazaaminopterin Compounds In Table IA Of Formula II Where A Is N

The following data illustrate administration to mice of several deazaaminopterin compounds of the invention and methotrexate in the evaluation of anti-inflammatory activity. The data are presented as two separate observations, the visually observed presence of inflammation in the mouse, and the caliper-measured degree of swelling of the rear paws of the mouse.

The efficacy evaluation used a mouse model of inflammatory disease that occurs in response to an antigenic challenge with Type II collagen [J. S. Courtenay et al., Nature, 283, 666-668 (1980)].

The fundamental aspects of the model allow it to serve as a representative presentation of human disease. The parallels between the known aspects of the mouse model and rheumatoid arthritis include a humoral response in which antibodies are produced to an antigen that is present in the joint tissue and the antigenic challenge is accompanied by cell-mediated aspects of immunity. The resultant inflammation of the joint tissue yields facets of periostitis, synovial lining hyperplasia, degradation of bone and cartilage and pannus and new bone formation.

The basic elements of the model included the immunization of DBA/1 mice with a suspension of fetal bovine Type II collagen (1 mg/ml) prepared in complete Freund's adjuvant. The primary injection was given using 0.1 ml of the collagen emulsion giving a total of 0.1 mg of Type II collagen per mouse. The animals were then given a booster injection of Type II collagen (100 µg in 0.01 M acetic acid ) on day 21 by intraperitoneal injection.

The results of the in vivo testing of methotrexate showed that using prophylactic regimens in which drug was begun two days prior to administration of antigen (Type II collagen) was more effective than starting drug at day 19, two days prior to the first and only boost with Type II collagen. Typically, in this model the untreated positive control animals have an incidence of arthritis ranging from 90 to 100% of injected animals at day 44.

The effect of methotrexate and test compounds on the extent of inflammation was determined by direct analysis of paw swelling using caliper measurements. The results are presented in Table IIA, and show a direct correlation between the decrease in the number of animals having disease and a decrease in the extent of inflammation, as determined by paw swelling.

**Table IIA**

| | **No Mice on day indicated**^{**b**} | | | | **Ave. thickness of rear paws (mm) over days 30-44**^{**c**} | |
|---|---|---|---|---|---|---|
| **Compound** | **Dose(mg/kg)** | **Day 30** | **Day 37** | **Day 44** | **Treated** | **Untreated** |
| None | | 31/43 | 38/43 | 41/43 | | |
| 5-Me-10-H (5-Me-5-DA) | 1.5 . | 0.8 | 2/8 | 6/8 | (2.18-2.55) | (2.33-2.87) |
| 5-Me-10-Me (5-Me-5-DMTX) | 1.5 | 1/8 | 4/8 | 5/8 | (2.26-2.44) | (2.33-2.87) |
| 5-Et-10-H (5-Et-5-DA) | 1.0 | 2/8 | 5/8 | 2/8 | (2.19-2.24) | (2.56-2.98) |
| 5-Et-10-Me (5-Et-5-DMTX) | 0.75 | 0/7 | 1/7 | 1/7 | (2.20-2,18) | (2.24-2.63) |
| 5-Pr-10-H (5-Pr-5-DA) | 1.5 | 0/7 | 0/7 | 0/7 | (2.14-2.15) | (2.24-2.63) |
| 5-H-10-Propargyl (10-Prgl-5-DA) | 12.0 | 3/8 | 2/8 | 6/8 | (2.22-2.37) | (2.34-2.78) |
| 5-Me-10-Propargyl (5-Me-10-Prgl-5-DA) | 1.5 | 3/8 | 3/8 | 2/8 | (2.29-2.29) | (2.56-2.98) |
| 5-Me-10-Allyl (5-Me-10-Allyl-5-DA) | 3.0 | 0/8 | 0/8 | 0/8 | (2.12-2.16) | (2.32-2.72) |
| MTX^{a} | 9.0 | 1/22 | 1/22 | 6/22 | (2.18-2.34) | (2.24-2.98) |

| | | | | | | |
|---|---|---|---|---|---|---|
| ^{a} Methotrexate (MTX) and untreated controls are composites from multiple runs. | | | | | | |
| ^{b} Visual evidence of inflammation. | | | | | | |
| ^{c} Values in parentheses are 30 day and 44 day measurements vs. equivalent for untreated controls; decrease in inflammation vs. control is most notable at day 44. | | | | | | |

It is apparent from the above results that the number of test mice affected was very considerably descreased by administration of deazaaminopterin compound. The results show that deazaaminopterin compound on a similar dosage level to be at least as effective as methotrexate, and since methotrexate is accepted as effective the deazaaminopterin compound is to be expected to be at least as effective as methotrexate, under similar conditions. The potent anti-arthritic activity of the deazaaminopterin compounds tested is evident from the results.

In addition to an effectiveness at least as great methotrexate at a similar dosage level, the compounds of the invention have an advantage in a lower-toxicity, meaning that dosages higher than methotrexate can be possible. The data in Table IIIA show less cytotoxicity on a human liver cell line (Chang liver) than methotrexate, in terms of the rates of cytotoxic potency versus methotrexate, whose ratio in those terms is 1.00. Thus, the higher the ratio is above 1, the lower the toxicity of the compound in respect to methotrexate.

**Table IIIA**

| **Ratio Of Cytotoxic Potency Versus Methotrexate For Inhibition Of Human Liver Cell Growth In Culture** | |
|---|---|
| **Compound** | **Ratio** |
| MTX | 1.00 |
| 5-Me-10-NH | 4.39 |
| 5-Me-10-NMe | 2.79 |
| 5-Et-10-NH | 4.50 |
| 5-Et-10-NMe | 3.18 |
| 5-Pr-10-NH | 1.78 |
| 5-Bu-10-NH | 7.20 |
| 5-Me-10-N-Propargyl | 21.18 |
| 5-H-10-N-Propargyl | 1.26 |
| 5-Me-10-N-allyl | |
| 5,10-Dideazaaminopterin | 2.46 |
| 10-Me-5,10-Dideazaaminopterin | 2.69 |

### Example 9: In Vivo Biology Of Type II Collagen Arthritis And Methotrexate Treatment IJsing 5-Deazaaminopterin Compounds In Table IA Of Formula II Wherein A Is N

The following illustrates administration to mice of several 5-deazaaminopterin compounds of the invention and methotrexate in the evaluation of anti-inflammatory activity. The data are presented in Table IVA below as two separate observations, the visually observed presence of inflammation in the mouse, and the visually observed presence of swelling of the rear paws of the mouse.

The efficacy evaluation uses a mouse model of inflammatory disease that occurs in response to an antigenic challenge with Type II collagen [J. S. Courtenay et al., Nature, 283, 666-668 (1980)], as described in Example 8 hereinabove.

**Table IVA**

| **Visual Observation Of Inflammation And Paw Swelling** | | | |
|---|---|---|---|
| | | **Visual Observation of** | |
| **Compound** | **Dose (mg/kg)** | **Inflammation** | **Presence of Swelling** |
| **None** | | | |
| 5-Me-10-H | 1.5 | Yes | Yes |
| 5,10-dideaza | | | |
| 5-Me-10-Me | 1.5 | Yes | Yes |
| 5,10-dideaza | | | |
| 5-Et-10-H | 1.0 | Yes | Yes |
| 5,10-dideaza | | | |
| 5-Et-10-Me | 0.75 | Yes | Yes |
| 5,10-dideaza | | | |
| 5-Pr-10-Me | 1.5 | Yes | Yes |
| 5,10-dideaza | | | |
| 5-H-10-Propargyl | 12.0 | Yes | Yes |
| 5,10-dideaza | | | |
| 5-Me-10-Propargyl | 1.5 | Yes | Yes |
| 5,10-dideaza | | | |
| 5-Me-10-Allyl 5,10-dideaza | 3.0 | Yes | Yes |

The results show the deazaaminopterin compound on a similar dosage level to be at least as effective as methotrexate, and since methotrexate is accepted as effective the deazaaminopterin compound is to be expected to be at least as effective as methotrexate, under similar conditions. The anti-arthritic activity of the deazaaminopterin compounds tested was confirmed by the results.

### Example 10: In Vivo Biology Of Type II Collagen Arthritis And Methotrexate Treatment Using Heteroaroyl-5-Deazaaminopterin Compounds Nos. A' To H' Of Table IB

The following data illustrate administration to mice of Compounds Nos. A' to D' of Table IB of the invention and methotrexate in the evaluation of anti-inflammatory activity. The data are presented as two separate observations, the visually observed presence of inflammation in the mouse, and the caliper-measured degree of swelling of the rear paws of the mouse.

The efficacy evaluation used a mouse model of inflammatory disease that occurs in response to an antigenic challenge with Type II collagen [Courtenay et al., nature, 283, 666-668 (1980)], as described in Example 8 hereinabove.

The effect of methotrexate and test compounds on the extent of inflammation was determined by direct analysis of paw swelling using caliper measurements. The results are presented in Table IIB, and show a direct correlation between the decrease in the number of animals having disease and a decrease in the extent of inflammation, as determined by paw swelling.

**Table IIB**

| | | No mice affected on day indicated^{b} | | | Avg. thickness of rear paws (mm) over days 30-44^{c} | |
|---|---|---|---|---|---|---|
| Compound | Dose (mg/kg) | Day 30 | Day 37 | Day 44 | Treated | Untreated |
| None | | 31/43 | 38/43 | 41/43 | | 2.29-2.73 |
| A' | | | | | | |
| B' | 6.0 | 0/5 | 2/5 | 2/5 | 2.19-2.33 | |
| C' | 6.0 | 0/8 | 1/8 | 3/8 | 2.13-2.27 | |
| D' | 6.0 | 2/8 | 4/8 | 7/8 | 2.13-2.32 | |
| MTX | 9.0 | 1/22 | 1/22 | 6/22 | 2.128-2.34 | |

| | | | | | | |
|---|---|---|---|---|---|---|
| a MTX and untreated controls are composites from multiple runs. b Visual evidence of inflammation. | | | | | | |
| c Values in parentheses are 30 day and 44 day measurements vs. equivalent for untreated controls; decrease in inflammation vs. control is most notable at day 44. | | | | | | |

It is apparent from the above results that the number of test mice affected was very considerably decreased by administration of heteroaroyl-5-deazaaminopterin or 5,10-dideazaaminopterin compound. The results show that heteroaroyl-5-deazaaminopterin or 5,10-dideazaaminopterin compound on a similar dosage level to be at least as effective as methotrexate, and since methotrexate is accepted as effective the heteroaroyl-5-deazaaminopterin or 5,10-dideazaaminopterin compound is to be expected to be at least as effective as methotrexate, under similar conditions. The potent anti-arthritic activity of the heteroaroyl-5-deazaaminopterin or 5,10-dideazaaminopterin compounds tested is evident from the results.

## Claims

1. 5-deazaaminopterin and 5,10-dideazaaminopterin compounds represented by the formula: 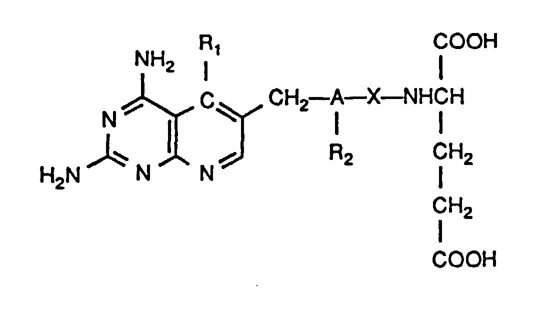 wherein
A is N;
X is one of 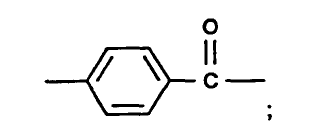 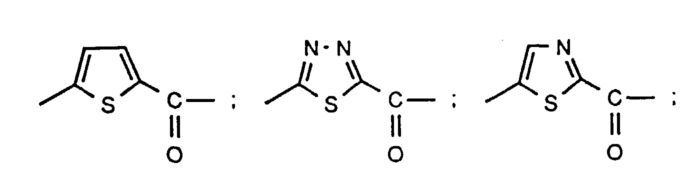 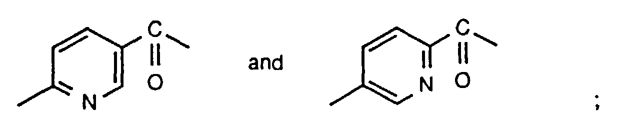 and R₁ and R₂ each represents hydrogen, or alkyl, alkenyl, or alkynyl group of up to eight carbon atoms;
provided that when X is 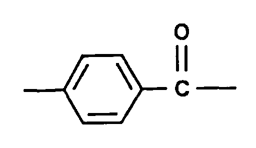 then R₁ is alkyl, alkenyl or alkynyl and when R₁ is alkyl, then R₂ is alkenyl or hydrogen provided R₁ is not methyl or ethyl when R₂ is hydrogen, and the pharmaceutically acceptable salts thereof.

2. Compounds or salts according to claim 1, wherein X is one of 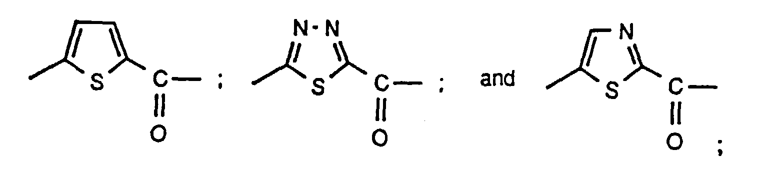 and R₁ is hydrogen, or an alkyl group having from one to eight carbon atoms.

3. Compounds or salts according to claim 1, wherein X is one of 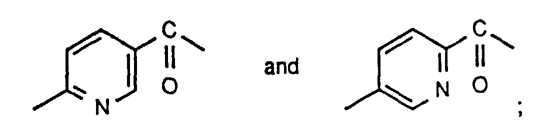 and R₁ is hydrogen, or an alkyl group having from one to eight carbon atoms.

4. Compounds or salts according to claim 2 or 3 wherein R₁ is hydrogen, or an alkyl group having from one to three carbon atoms, and R₂ is hydrogen, an alkyl group of 1 to 3 carbon atoms or an alkenyl, or alkynyl group of up to three carbon atoms.

5. Compounds or salts according to claim 1, wherein X is 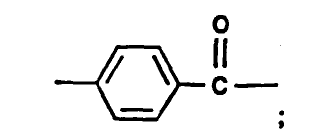 at least one of R₁ and R₂ is an alkyl, alkenyl or alkynyl group of from three to five carbon atoms.

6. Compounds or salts according to claim 5, wherein at least one of R₁ and R₂ is an alkyl group having from three to five carbon atoms.

7. Compounds or salts according to claim 1, wherein X is 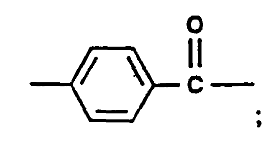 and at least one of R₁ and R₂ is alkenyl.

8. Use of a 5-deazaaminopterin or 5, 10-dideazaaminopterin of the formula 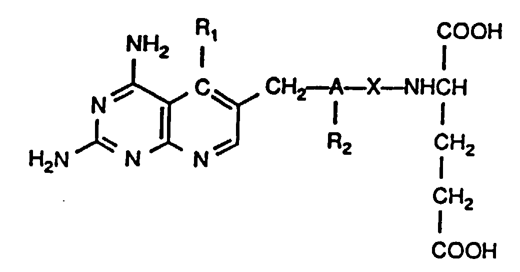 where
A is N;
X is one of 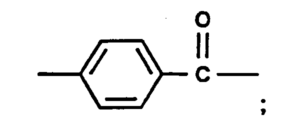 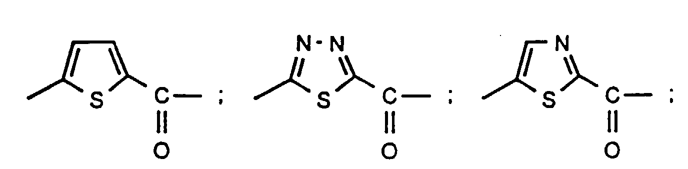 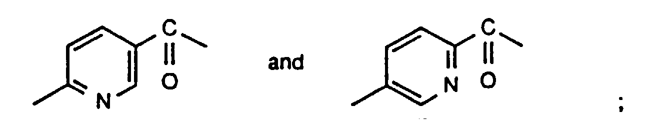 and R₁ and R₂ each represents hydrogen, or alkyl, alkenyl, or alkynyl group of up to eight carbon atoms; or a pharmaceutically acceptable salf thereof, for the manufacture of a medicament for use in the treatment of an inflammatory disease.

9. Use according to claim 8. wherein the inflammatory disease is arthritis.

10. Use according to claim 8 or 9, wherein the inflammatory disease is arthritis,
wherein X is 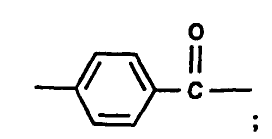 and at least one of R₁ and R₂ is an alkyl or alkenyl group having from three to five carbon atoms;
or where X is 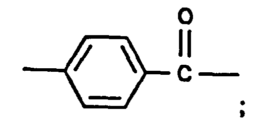 and one of R₁ and R₂ is alkyl and the other of R₁ and R₂ is alkenyl or alkynyl.

11. A use in accordance with claim 8 or 9 wherein the medicament is in the form of a pharmaceutical composition in unit dosage form and containing from 0.1 to 500 mg of said compound or salt per unit dose.

## Patentansprüche

1. 5-Deazaaminopterin- und 5,10-Dideazaaminopterinverbindungen der Formel 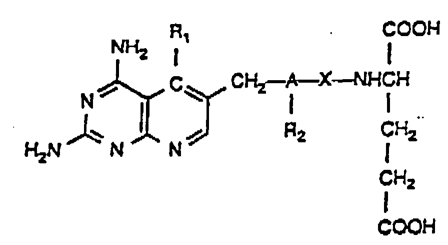 worin A N ist, X eine der Gruppen 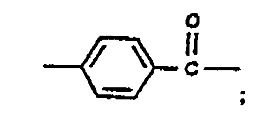 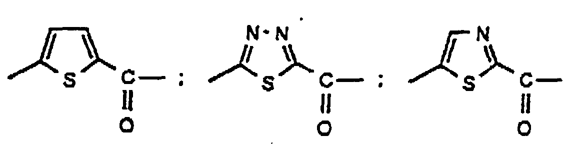 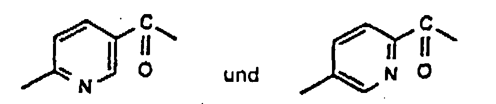 ist, und R₁ und R₂ jeweils Wasserstoff oder eine Alkyl-, Alkenyl- oder Alkinylgruppe mit bis zu 8 Kohlenstoffatomen bedeuten,
wobei, wenn X 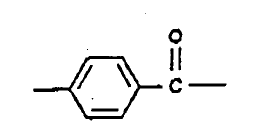 ist, R₁ Alkyl, Alkenyl oder Alkinyl ist, und wenn R₁ Alkyl ist, R₂ Alkenyl oder Wasserstoff ist, vorausgesetzt, daß R₁ nicht Methyl oder Ethyl ist, wenn R₂ Wasserstoff ist. und deren pharmazeutisch verträgliche Salze.

2. Verbindungen oder Salze nach Anspruch 1, worin X eine der Gruppen 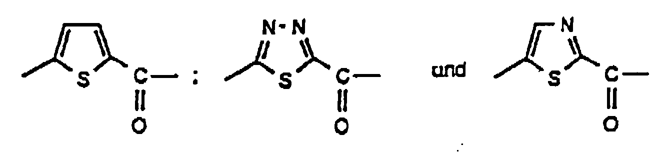 bedeutet und R₁ Wasserstoff oder eine Alkylgruppe mit 1 bis Kohlenstoffatomen ist.

3. Verbindungen oder Salze nach Anspruch 1, worin X eine der Gruppen 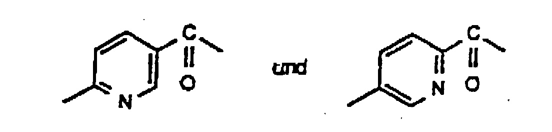 ist und R₁ Wasserstoff oder eine Alkylgruppe mit 1 bis 8 Kohlenstoffatomen ist.

4. Verbindungen oder Salze nach Anspruch 2 oder 3, worin R₁ Wasserstoff oder eine Alkylgruppe mit 1 bis 3 Kohlenstoffatomen ist und R₂ Wasserstoff, eine Alkylgruppe mit 1 bis 3 Kohlenstoffatomen oder eine Alkenyl- oder Alkinylgruppe mit bis zu 3 Kohlenstofiatomen ist.

5. Verbindungen oder Salze nach Anspruch 1, worin X 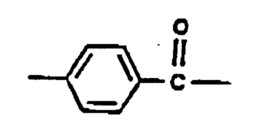 ist und wenigstens einer der Reste R₁ und R₂ eine Alkyl-, Alkenyl- oder Alkinylgruppe mit 3 bis 5 Kahlenstoffatomen ist.

6. Verbindungen oder Salze nach Anspruch 5, worin wenigstens einer der Reste R₁ und R₂ eine Alkylgruppe mit 3 bis 5 Kohlenstoffatomen ist.

7. Verbindungen oder Salze nach Anspruch 1, worin X 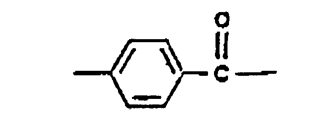 ist und wenigstens einer der Reste R₁ und R₂ Alkenyl ist.

8. Verwendung eines 5-Deazaaminopterins oder 5,10-Dideazaaminopterins der Formel 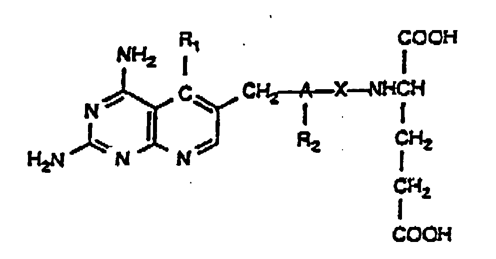 worin A N ist, X eine der Gruppen 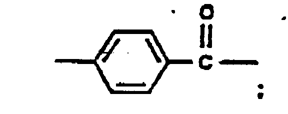 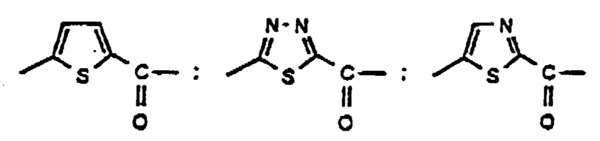 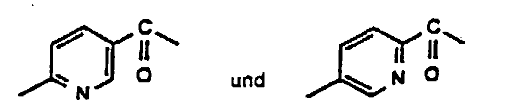 ist und R₁ und R₂ jeweils Wasserstoff oder eine Alkyl-, Alkenyl- oder Alkinylgruppe mit bis zu 8 Kohlenstoffatomen bedeutet, oder eines pharmazeutisch verträglichen Salze hiervon für die Herstellung eines Arzneimittels für die Verwendung bei der Behandlung einer entzündlichen Erkrankung.

9. Verwendung nach Anspruch 8, bei der die entzündliche Erkrankung Arthritis ist.

10. Verwendung nach Anspruch 8 oder 9, bei der die entzündliche Erkrankung Arthritis ist, wobei X 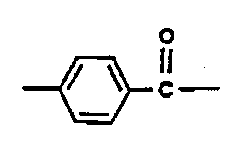 ist und wenigstens einer der Reste R₁ und R₂ eine Alkyl- oder Alkenylgruppe mit 3 bis 5 Kohlenstoffatomen ist, oder X 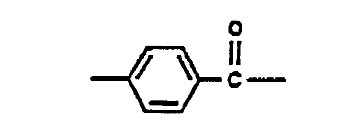 ist und einer der Reste R₁ und R₂ Alkyl und der andere der Reste R₁ und R₂ Alkenyl oder Alkinyl ist.

11. Verwendung nach Anspruch 8 oder 9, bei der das Arzneimittel in der Form einer pharmazeutischen Zusammensetzung in Dosielrungseinheitsform vorliegt und 0,1 bis 500 mg der Verbindung oder des Salzes je Dosierungseinheit enthält.

## Revendications

1. Dérivés de 5-déazaaminoptérine et 5,10-didéazaaminoptérine représentés par la formule : 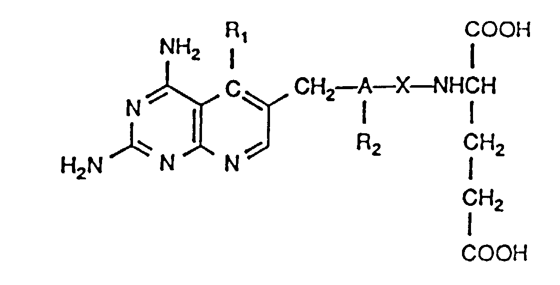 dans laquelle
A représente N ;
X représente un des groupes de formules 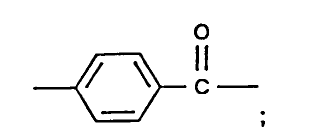 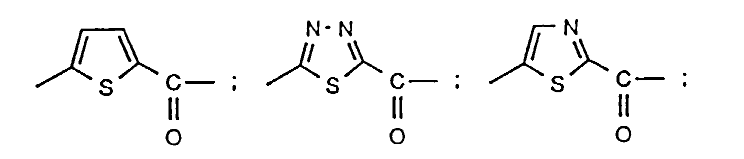 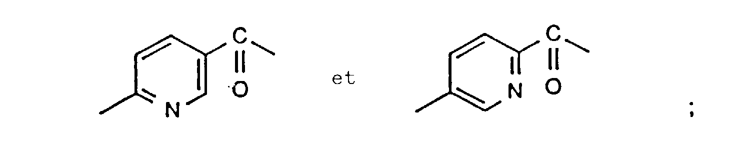 et R₁ et R₂ représentent chacun l'hydrogène ou un groupe alkyle, alcényle, ou alcynyle ayant jusqu'à 8 atomes de carbone ;
sous réserve que,
lorsque X représente un groupe 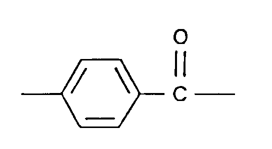 alors R₁ représente un groupe alkyle, alcényle ou alcynyle et, lorsque R₁ représente un groupe alkyle, alors R₂ représente un groupe alcényle ou l'hydrogène, sous réserve que R₁ ne représente pas un groupe méthyle ou éthyle lorsque R₂ représente l'hydrogène, et leurs sels pharmaceutiquement acceptables.

2. Composés ou sels suivant la revendication 1, dans lesquels X représente un des groupes de formules 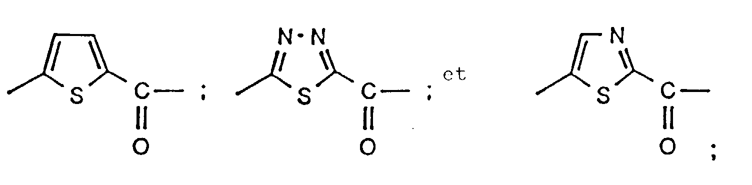 et R₁ représente l'hydrogène ou un groupe alkyle ayant 1 à 8 atomes de carbone.

3. Composés ou sels suivant la revendication 1, dans lesquels X représente un des groupes de formules 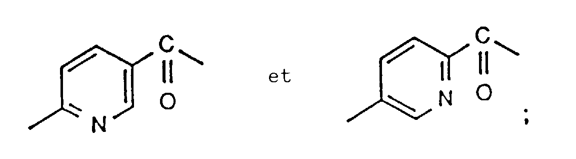 et R₁ représente l'hydrogène ou un groupe alkyle ayant 1 à 8 atomes de carbone.

4. Composés ou sels suivant la revendication 2 ou 3, dans lesquels R₁ représente l'hydrogène ou un groupe alkyle ayant 1 à 3 atomes de carbone, et R₂ représente l'hydrogène, un groupe alkyle ayant 1 à 3 atomes de carbone ou un groupe alcényle ou alcynyle ayant jusqu'à 3 atomes de carbone.

5. Composés ou sels suivant la revendication 1, dans lesquels X représente un groupe 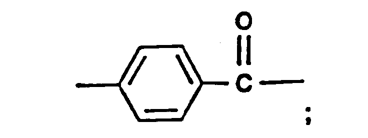 au moins un des groupes R₁ et R₂ représente un groupe alkyle, alcényle ou alcynyle ayant 3 à 5 atomes de carbones.

6. Composés ou sels suivant la revendication 5, dans lesquels au moins un des groupes R₁ et R₂ représente un groupe alkyle ayant 3 à 5 atomes de carbone.

7. Composés ou sels suivant la revendication 1, dans lesquels X représente un groupe 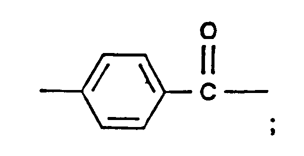 et au moins un des groupes R₁ et R₂ représente un groupe alcényle.

8. Utilisation d'une 5-déazaaminoptérine ou 5,10-didéazaaminoptérine, de formule 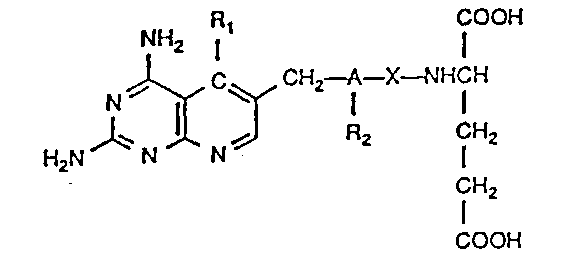 dans laquelle
A représente N ;
X représente un des groupes de formules 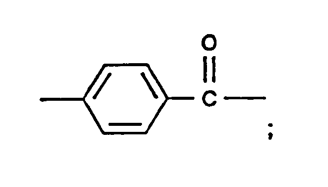 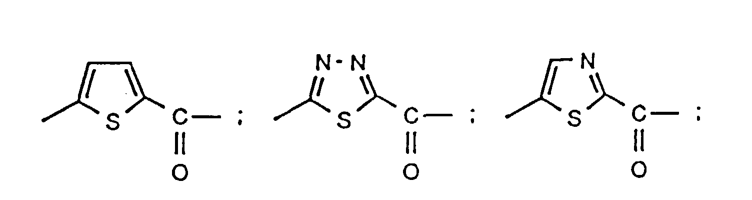 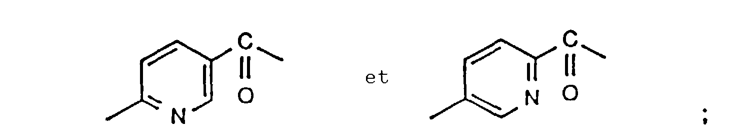 et R₁ et R₂ représentent chacun l'hydrogène ou un groupe alkyle, alcényle ou alcynyle ayant jusqu'à 8 atomes de carbone ;
ou d'un de ses sels pharmaceutiquement acceptables, pour la production d'un médicament destiné à être utilisé dans le traitement d'une maladie inflammatoire.

9. Utilisation suivant la revendication 8, dans laquelle la maladie inflammatoire est l'arthrite.

10. Utilisation suivant la revendication 8 ou 9, dans laquelle la maladie inflammatoire est l'arthrite, X représente un groupe 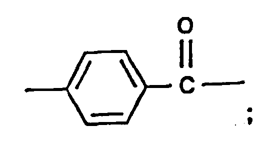 et au moins un des groupes R₁ et R₂ représente un groupe alkyle ou alcényle ayant trois à cinq atomes de carbone ;
ou bien X représente un groupe 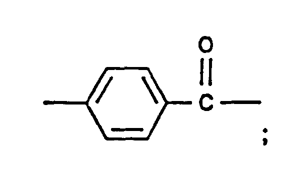 et un des groupes R₁ et R₂ représente un groupe alkyle et l'autre des groupes R₁ et R₂ représente un groupe alcényle ou alcynyle.

11. Utilisation suivant la revendication 8 ou 9, dans laquelle le médicament est sous forme d'une composition pharmaceutique sous une forme posologique unitaire contenant 0,1 à 500 mg dudit composé ou sel par dose unitaire.
